(19) Europäisches Patentamt
European Patent Office
Office européen des brevets

(11) **EP 2 379 227 B1**

(12) **EUROPEAN PATENT SPECIFICATION**

(45) Date of publication and mention
of the grant of the patent:
**22.01.2014 Bulletin 2014/04**

(51) Int Cl.:
*C12M 1/00* *(2006.01)*    *G01N 33/18* *(2006.01)*
*G01N 1/40* *(2006.01)*    *C12Q 1/24* *(2006.01)*

(21) Application number: **09797243.4**

(22) Date of filing: **30.12.2009**

(86) International application number:
**PCT/US2009/069785**

(87) International publication number:
**WO 2010/078404 (08.07.2010 Gazette 2010/27)**

(54) **METHODS FOR ISOLATING MICROORGANISMS**

VERFAHREN ZUR ISOLIERUNG VON MICROORGANISMEN

MÉTHODES D'ISOLEMENT DE MCIRO-ORGANISMES

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: **31.12.2008 US 141813 P**

(43) Date of publication of application:
**26.10.2011 Bulletin 2011/43**

(73) Proprietor: **3M Innovative Properties Company**
**Saint Paul, MN 55133-3427 (US)**

(72) Inventors:
• **KSHIRSAGAR, Manjiri T.**
**Saint Paul, Minnesota 55133-3427 (US)**

• **HALVERSON, Kurt J.**
**Saint Paul, Minnesota 55133-3427 (US)**
• **RAJAGOPAL, Raj**
**Saint Paul, Minnesota 55133-3427 (US)**

(74) Representative: **Vossius & Partner**
**Siebertstrasse 4**
**81675 München (DE)**

(56) References cited:
**WO-A1-89/09279        WO-A1-2008/150779**
**WO-A1-2009/067503      WO-A1-2009/067518**
**WO-A2-2007/137257**

**Description**

The present disclosure relates to methods for processing samples for microorganism analysis.

The determination of the absence or presence of coliform bacteria (as well as other microorganisms and contaminants) is one of the basic analyses to assess the sanitary quality of water. The presence of fecal coliforms in a sample is a primary indication of fecal contamination of the sample water and may indicate the possible presence of other pathogenic organisms. Methods for enumerating microbes in water samples are well known and many have been standardized.

WO 89/09279 A1 discloses a method for collecting, concentrating and detecting microorganisms from difficult-to-separate environmental samples e.g. oil well samples and the like, for the purpose of their analysis or identification; and apparatus for performing the method. The method comprises the following steps: mixing a fluid sample with particles of a sorbent that adsorbs or entraps cells of the microorganism; agitating said mixture to provide a substantially uniform suspension of sorbent particles, microorganisms and other solid impurities in said fluid; passing said suspension through a filter that retains substantially solely the sorbent particles containing the entrapped or adsorbed microorganisms as a cake; optionally, washing said cake of substantially all soluble impurities; re-suspending said sorbent particles containing microorganisms in a pure liquid substantially inert to said microorganisms, wherein said sorbent particles separate from said microorganisms; and permitting said sorbent particles to settle thus releasing said microorganisms into the supernatant liquid.

WO 2008/150779 A1 discloses manual devices for collecting and concentrating liquid samples for microbiological analysis and their respective methods of use. The manual devices comprise a body which contains a sample, a removable support, a microporous membrane, and a plunger.

Currently available standard methods for enumerating bacteria in water samples are generally expensive and require multiple steps, sophisticated instrumentation and highly trained personnel.

Membrane filtration is a commonly utilized technique to obtain a direct count of microorganisms present in low concentrations in large volume samples of water. The operational requirements for most membrane filtration techniques (vacuum manifolds) or centrifugation (powered equipment) make them unsuitable for on-site applications. In addition, filtration of large volumes by use of mechanical means (via pistons or plungers) or manually applied pressure is very labor intensive due to the pressure required to force the sample through membranes having pore sizes small enough to isolate bacteria (0.22 to 0.45 microns). Thus, there is a need for simple, non-labor intensive, inexpensive, portable sample acquisition methods for processing large sample volumes.

The present invention relates to a method for isolating microorganisms according to claims 1 and 7.

Disclosed herein is a kit (not forming part of the invention) that includes concentration agent; and a system for isolating microorganisms from a sample, the system including: a receptacle configured to allow filtering of the sample and to reversibly contain the sample and a concentration agent; and a filter, the filter having a first surface and a second surface and comprising pores having an average pore size that is larger than the average size of the microorganisms.

Disclosed herein is a system (not forming part of the invention) for isolating microorganisms from a sample, the sample having sample matrix and microorganisms, the system including a liner configured to afford contact of a concentration agent and the sample, to provide a microorganism-bound composition that includes concentration agent-bound microorganisms and sample matrix; a filter, the filter having a first surface and a second surface and comprising pores having an average pore size that is larger than the average size of the microorganisms; a filter support configured to contact the first surface of the filter and afford contact of the microorganism-bound composition with the second surface of the filter, wherein the liner and filter support are configured to afford filtration of the microorganism-bound composition through the filter in order to collect the concentration agent-bound microorganisms on the second surface of the filter.

Disclosed herein is a kit (not forming part of the invention) that includes concentration agent; and a system for isolating microorganisms from a sample, the system including: a liner configured to afford contact of the concentration agent and the sample, providing a microorganism-bound composition that includes concentration agent-bound microorganisms and sample matrix; a filter, the filter having a first surface and a second surface and having pores having an average pore size that is larger than the average size of the microorganisms; a filter support configured to contact the first surface of the filter and  afford contact of the microorganism-bound composition with the second surface of the filter, wherein the liner and filter support are configured to afford filtration of the microorganism-bound composition through the filter in order to collect the concentration agent having bound microorganisms on the second surface of the filter.

Disclosed herein is a method (not forming part of the invention) for isolating microorganisms from a sample, the sample having sample matrix and microorganisms, the method including the steps of: placing a liner having an opening in a container to form a receptacle assembly, the container configured to at least partially receive the liner; adding the sample to the liner to form a microorganism-bound composition in the liner, the microorganism-bound composition including concentration agent-bound microorganisms and sample matrix; placing a filter over at least a portion of the opening of the liner; placing a filter support on the filter to form a filter assembly, the filter assembly including the liner, the container, the filter and the filter support; inverting the filter assembly; and filtering the microorganism-bound composition through the filter to collect the concentration agent-bound microorganisms on the filter.

The disclosure may be more completely understood in consideration of the following detailed description of various embodiments of the disclosure in connection with the accompanying drawings, in which:

The figures are not necessarily to scale. Like numbers used in the figures refer to like components. However, it will be understood that the use of a number to refer to a component in a given figure is not intended to limit the component in another figure labeled with the same number.

**FIG. 1** illustrates an exemplary method disclosed herein;
**FIG. 2** illustrates another exemplary method disclosed herein;
**FIG. 3** illustrates another exemplary method disclosed herein;
**FIG. 4** illustrates another exemplary method disclosed herein;
**FIG. 5** illustrates another exemplary method disclosed herein;
**FIG. 6** illustrates an exemplary kit as disclosed herein;
**FIGs. 7A** and **7B** are exploded perspective **(FIG. 7A)** and top **(FIG. 7B)** views of an exemplary device that can be utilized in a method, kit or system as described herein;
**FIGs. 8A - 8F** are exploded **(FIGs. 8A** and **8B),** schematic **(FIG. 8C),** cross-sectional **(FIG. 8D),** top view **(FIG. 8E)** and perspective **(FIG. 8F)** views of an exemplary device that can be utilized in a method, kit or system as described herein; and
**FIGs. 9A - 9C** are exploded **(FIG. 9A),** cross-sectional **(FIG. 9B)** and top **(FIG. 9C)** views of an exemplary device that can be utilized in a method, kit or system as described herein.

In the following description, reference is made to the accompanying drawings that form a part hereof, and in which are shown by way of illustration several specific aspects. It is to be understood that other aspects are contemplated and may be made without departing from the scope of the present disclosure. The following detailed description, therefore, is not to be taken in a limiting sense.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

The recitation of numerical ranges by endpoints includes all numbers subsumed within that range (e.g. 1 to 5 includes 1, 1.5, 2, 2.75, 3, 3.80, 4, and 5) and any range within that range.

As used in this specification and the appended claims, the singular forms "a", "an", and "the" encompass aspects having plural referents, unless the content clearly dictates otherwise. As used in this specification and the appended claims, the term "or" is generally employed in its sense including "and/or" unless the content clearly dictates otherwise.

Disclosed herein are methods, systems, kits and devices for processing samples. The methods, systems, kits and devices can offer advantages in processing samples in that they provide a quick, easy, simple and relatively inexpensive way to prepare water samples (for example) for microbiological analyses. Further advantage is provided by the ability to accomplish universal sample concentration options from large volumes for detecting low levels of bacteria, spores or viruses. The methods and devices can significantly reduce back pressure and leakage issues that are often associated with filtering for microbiological analyses (because of the very small pore size filters that are usually required). The methods and devices can be quick, simple, portable and require no expensive equipment or highly skilled technician. When large pore size filters (e.g. at least about 10 $\mu$m or greater) are utilized, coliform enumeration can be done directly in commercially available culture films, such as 3M™ PETRIFILM™ E.Coli/Coliform Count Plates (3M Company, St. Paul MN). When utilized with disposable receptacles, only the disposable portion of the system contacts the sample, thereby eliminating the need to clean and sterilize the device prior to the next use.

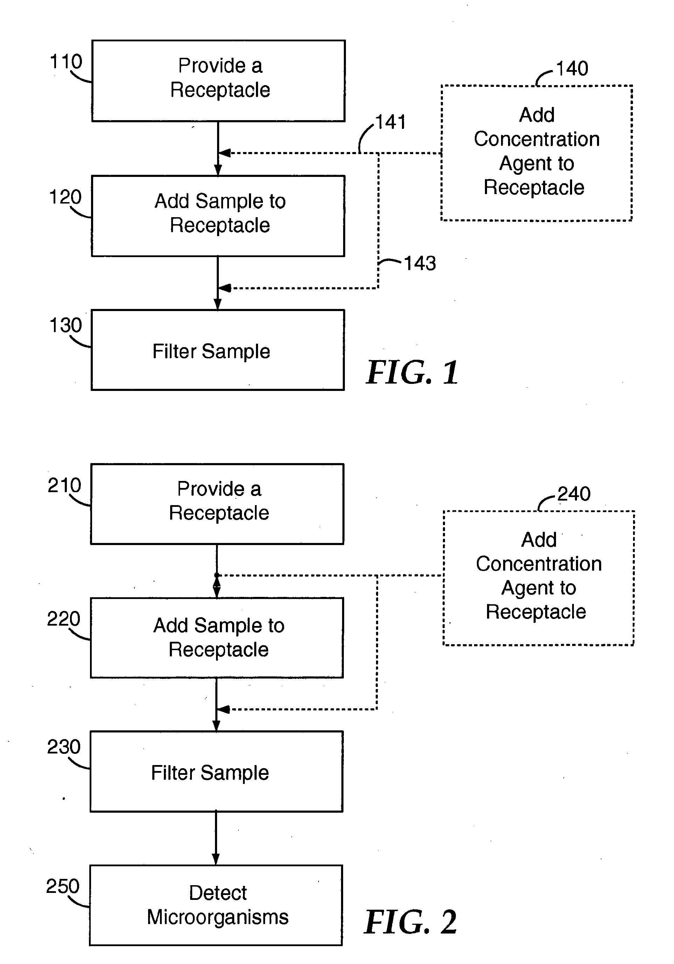

An exemplary method is depicted in **FIG. 1.** The method depicted in **FIG. 1** includes step **110,** providing a receptacle; step **120,** adding sample to the receptacle; and step **130,** filtering the sample.

The first step in the exemplary method, step **110** includes providing a receptacle. The step of providing a receptacle can include merely obtaining a receptacle or making a receptacle. For example, a receptacle could be purchased for use in the method, a receptacle could be modified from a purchased article, or a receptacle could be made based on the teaching provided herein, for example.

Generally, a receptacle that can be utilized herein is configured to at least reversibly contain the sample and allow filtering of the sample. Numerous types and configurations of receptacles could be utilized in the method and kits disclosed herein. A receptacle can be a single opening receptacle or a multiple opening receptacle. A single opening receptacle can be configured to afford addition of the sample (and other optional components) and filtering of the sample from the same opening. A multiple opening receptacle can be configured to afford addition of the sample (and other optional components) via one opening and filtering of the sample via a second opening. In an example, a multiple opening receptacle includes two openings and is referred to herein as a double opening receptacle. The application can dictate the type of receptacle to be utilized.

A receptacle that can be utilized herein can be formed of a variety of materials including, but not limited to, polymeric materials, metals (e.g., aluminum, stainless steel, etc.), ceramics, glasses, and combinations thereof. Examples of polymeric materials can include, but are not limited to, polyolefins (e.g., polyethylene, polypropylene, combinations thereof, etc.), polycarbonate, acrylics, polystyrene, high density polyethylene (HDPE), polypropylene, other suitable polymeric materials capable of forming a freestanding and/or self-supporting container, or a combination thereof. The receptacle can be made of materials which are relatively inexpensive and can therefore be disposable. The receptacle can be translucent (or even transparent), or opaque, and can be any suitable size, depending on the type, amount and size of source to be analyzed. The application can dictate the amount of sample to be utilized. The receptacle can have a capacity of any useful volume, for example, a capacity from about 5 mL to about 1000 mL. The receptacle can have a capacity from about 5 mL to about 500 mL. The receptacle can have a capacity from about 5 mL to about 250 mL. The receptacle can have a capacity from about 10 mL to about 250 mL. For example, the receptacle can have a capacity of 10 mL, 50 mL, 100 mL, 250 mL, or larger for example.

A receptacle that can be utilized herein is configured to at least reversibly contain the sample. Reversibly contain the sample means that the receptacle can contain the sample (and other components) but the sample or at least a portion of the sample can be removed from the receptacle by for example, filtering. The size of receptacle to be utilized can therefore depend at least in part on the size of the sample to be collected. The sample can be processed (i.e. combined with concentration agent) in a different container and can then be filtered utilizing the receptacle. The receptacle can contain a larger volume or smaller volume than the sample to be collected. The receptacle can be chosen based on the sample size to be collected, or the sample size to be collected can be chosen based on the receptacle size.

A receptacle that can be utilized herein is configured to allow the sample to be filtered. Generally, this implies that the receptacle is configured to be operably coupled with a filter. The receptacle can contain or can be configured to be coupled with an element that supports a filter. This element can be referred to as a filter support. A filter support can function to maintain the filter in operable communication with the receptacle. Exemplary types of filter supports can offer support across substantially the entire surface area of the filter or less than the entire surface area of the filter. The combination of the filter, the receptacle and the filter support can be referred to herein as a filter assembly. In an example with a disposable liner and a container that holds the disposable liner (discussed in greater detail below), the combination of the filter, the container, the liner and the filter support can be referred to as a filter assembly.

Specific exemplary types of receptacles can be found in the following commonly assigned Patent Applications; PCT Publication No. WO2009/067503, entitled "SAMPLE PREPARATION FOR ENVIRONMENTAL SAMPLING"; PCT Patent Publication No. WO2007/137257, entitled "SYSTEM AND METHOD FOR PREPARING SAMPLES"; and PCT Publication No. WO2008/150779, entitled "DEVICES AND PROCESSES FOR COLLECTING AND CONCENTRATING SAMPLES FOR MICROBIOLOGICAL ANALYSIS". Further details related to some of the receptacle types disclosed in these applications will be discussed below.

The second step in the exemplary method depicted in **FIG. 1** is step **120,** adding a sample to the receptacle.

Generally, a sample contains sample matrix and microorganisms. The term sample matrix generally refers to everything within a sample besides the microorganisms. For example, the sample matrix in a water sample includes water, the dissolved material in the water sample and the undissolved material (with the exception of the microorganisms).

The term "microorganism" is generally used to refer to any prokaryotic or eukaryotic microscopic organism, including without limitation, one or more of bacteria (e.g., motile or vegetative, Gram positive or Gram negative), viruses (e.g., *Norovirus, Norwalk virus, Rotavirus, Adenovirus,* DNA viruses, RNA viruses, enveloped, non-enveloped, human immunodeficiency virus (HIV), human *Papillomavirus* (HPV), etc.), bacterial spores or endospores, algae, fungi (e.g., yeast, filamentous fungi, fungal spores), prions, mycoplasmas, and protozoa. In some cases, the microorganisms of particular interest are those that are pathogenic, and the term "pathogen" is used to refer to any pathogenic microorganism. Examples of pathogens can include, but are not limited to, members of the family *Enterobacteriaceae,* or members of the family *Micrococaceae,* or the genera *Staphylococcus* spp., *Streptococcus,* spp., *Pseudomonas* spp., *Enterococcus* spp., *Salmonella* spp., *Legionella* spp., *Shigella* spp., *Yersinia* spp., *Enterobacter* spp., *Escherichia* spp., *Bacillus* spp., *Listeria* spp., *Campylobacter* spp., *Acinetobacter* spp., *Vibrio* spp., *Clostridium* spp., and *Corynebacteria* spp. Particular examples of pathogens can include, but are not limited to, *Escherichia coli* including enterohemorrhagic *E. coli* e.g., serotype O157:H7, *Pseudomonas aeruginosa, Bacillus cereus, Bacillus anthracis, Salmonella enteritidis, Salmonella typhimurium, Listeria monocytogenes, Clostridium botulinum, Clostridium perfringens, Staphylococcus aureus,* methicillin-resistant *Staphylococcus aureus, Campylobacter jejuni, Yersinia enterocolitica, Vibrio vulnificus, Clostridium difficile,* vancomycin-resistant *Enterococcus,* and *Enterobacter sakazakii.* Environmental factors that may affect the growth of a microorganism can include the presence or absence of nutrients, pH, moisture content, oxidation-reduction potential, antimicrobial compounds, temperature, atmospheric gas composition and biological structures or barriers.

The amount of sample added to the receptacle can depend at least in part on the size and configuration of the receptacle, the amount of sample to be tested, other factors not discussed herein, or a combination thereof. The amount of sample to be added to the receptacle can be between about 5 mL and 1000 mL. The amount of sample to be added to the receptacle can be between about 5 mL and 500 mL. The amount of sample to be added to the receptacle can be between about 10 mL and about 250 mL. Addition of the sample to the receptacle can be accomplished by any known method, including but not limited to, pouring or otherwise adding the sample (from another container) to the receptacle and immersing at least a portion of the receptacle into a larger portion of the sample (e.g. utilizing the receptacle to obtain a sample from a water supply for example).

One function of the receptacle is to allow the sample including microorganisms to interact with a concentration agent. A concentration agent is generally a material that microorganisms will bind to when the microorganisms are dispersed in a sample matrix. A concentration agent can be a particulate material or can be a dispersed material. Suitable particulate or dispersed materials include, for example, metal carbonates (e.g., calcium carbonate) and metal phosphates (e.g., hydroxyapatite). Binding of microorganisms by such concentration agents is generally not specific to any particular strain, species, or type of microorganism and therefore provides for the concentration of a general population of microorganisms in a sample. Specific strains of microorganisms can then be detected from among the captured microorganism population using any known detection method for example with strain-specific probes or with strain-specific culture media. Once the sample is combined with the concentration agent in the receptacle, a microorganism-bound composition is obtained. A microorganism-bound composition includes concentration agent-bound microorganisms and sample matrix.

One exemplary type of concentration agents include diatomaceous earth and surface-treated diatomaceous earth. Specific examples of such concentration agents can be found in commonly assigned PCT Patent Publication No. WO2009/046191, entitled "MICROORGANISMS CONCENTRATION PROCESS AND AGENT". When dispersed or suspended in water systems, inorganic materials exhibit surface charges that are characteristic of the material and the pH of the water system. The potential across the material-water interface is called the "zeta potential," which can be calculated from electrophoretic mobilities (that is, from the rates at which the particles of material travel between charged electrodes placed in the water system). Concentration agents can have zeta potentials that are at least somewhat more positive than that of untreated diatomaceous earth, and the concentration agents can be surprisingly significantly more effective than untreated diatomaceous earth in concentrating microorganisms such as bacteria, the surfaces of which generally tend to be negatively charged.

One exemplary type of concentration agent includes diatomaceous earth. Another exemplary type of concentration agent includes surface-treated diatomaceous earth. Exemplary surface treatment includes a surface modifier, such as titanium dioxide, fine-nanoscale gold or platinum, or a combination thereof. Such surface treatments can be surprisingly more effective than untreated diatomaceous earth in concentrating microorganisms. The surface treatment can also further include a metal oxide selected from ferric oxide, zinc oxide, aluminum oxide, and the like, and combinations thereof. In an example, ferric oxide is utilized. Although noble metals such as gold have been known to exhibit antimicrobial characteristics, the gold-containing concentration agents can be effective not only in binding the microorganisms but also in leaving them viable for purposes of detection or assay.

Useful surface modifiers include fine-nanoscale gold; fine-nanoscale platinum; fine-nanoscale gold in combination with at least one metal oxide (for example, titanium dioxide, ferric oxide, or a combination thereof); titanium dioxide; titanium dioxide in combination with at least one other (that is, other than titanium dioxide) metal oxide; and the like; and combinations thereof. Surface modifiers such as fine-nanoscale gold; fine-nanoscale platinum; fine-nanoscale gold in combination with at least ferric oxide or titanium dioxide; titanium dioxide; titanium dioxide in combination with at least ferric oxide; or combinations thereof can be utilized.

Surface modifiers such as the following can be utilized: fine-nanoscale gold; fine-nanoscale platinum; fine-nanoscale gold in combination with ferric oxide or titanium dioxide; titanium dioxide; titanium dioxide in combination with ferric oxide; and combinations thereof. Fine-nanoscale gold; fine-nanoscale gold in combination with ferric oxide or titanium dioxide; titanium dioxide in combination with ferric oxide; and combinations thereof can be utilized. Fine-nanoscale gold, fine-nanoscale gold in combination with ferric oxide or titanium dioxide, and combinations thereof can also be utilized in an embodiment.

Another exemplary type of concentration agent includes gamma-FeO(OH) (also known as lepidocrocite). Specific examples of such concentration agents can be found in commonly assigned PCT Patent Publication No. WO2009/046183, entitled "MICROORGANISM CONCENTRATION PROCESS". Such concentration agents have been found to be surprisingly more effective than other iron-containing concentration agents in capturing gram-negative bacteria, which can be of great concern in regard to food- and waterborne illnesses and human bacterial infections. The concentration agents can further include (in addition to gamma-FeO(OH)) other components (for example, boehmite ($\alpha$-AlO(OH)), clays, iron oxides, and silicon oxides). In examples where such other components are included, they generally do not significantly interfere with the intimate contact of the sample and the concentration agent.

Gamma-FeO(OH) is known and can be chemically synthesized by known methods (for example, by oxidation of ferrous hydroxide at neutral or slightly acidic pHs, as described for purposes of magnetic tape production in U.S. Patent No. 4,729, 846 (Matsui et al.). Gamma-FeO(OH) is also commercially available (for example, from Alfa Aesar, A Johnson Matthey Company, Ward Hill, MA, and from Sigma-Aldrich Corporation, St. Louis, MO).

In an example that utilized gamma-FeO(OH) as a concentration agent, the gamma-FeO(OH) is generally in the form of microparticles. In an example, it is in the form of microparticles having particle sizes (largest dimension) in the range of about 3 micrometers (in other embodiments, about 5 micrometers; or about 10 micrometers) to about 100 micrometers (in other embodiments, about 80 micrometers; or about 50 micrometers; or about 35 micrometers; where any lower limit can be paired with any upper limit of the range). In an example, the particles are agglomerates of smaller particles. The particles can include crystallites that are less than about 1 micrometer in size (in an embodiment, less than about 0.5 micrometer in size). The crystallites can be present as acicular crystallites, as raft-like structures comprising acicular crystallites, or as combinations of the acicular crystallites and raft-like structures.

In an example, the concentration agents have a surface area as measured by the BET (Brunauer-Emmett-Teller) method (calculation of the surface area of solids by physical adsorption of nitrogen gas molecules) that is greater than about 25 square meters per gram ($m^2$/g); in an embodiment greater than about 50 $m^2$/g; and in another embodiment greater than about 75 $m^2$/g.

An agglomerated form of the particles can provide the adsorptive capabilities of fine particle systems without the handling and other hazards often associated with fine particles. In addition, such agglomerate particles can settle readily in fluid and thus can provide rapid separation of microorganisms from a fluid phase (as well as allowing low back pressure when filtered).

Another exemplary type of concentration agents include metal silicates. Specific examples of such concentration agents can be found in commonly assigned PCT Patent Publication No. WO2009/085357, entitled "MICROORGANISM CONCENTRATION PROCESS". Exemplary metal silicates can have a surface composition having a metal atom to silicon atom ratio of less than or equal to about 0.5 (in an embodiment, less than or equal to about 0.4; in another embodiment, less than or equal to about 0.3; in yet another embodiment, less than or equal to about 0.2), as determined by X-ray photoelectron spectroscopy (XPS). The surface composition may also include at least about 10 average atomic percent carbon (in an embodiment, at least about 12 average atomic percent carbon; in yet another embodiment at least about 14 average atomic percent carbon), as determined by X-ray photoelectron spectroscopy (XPS). XPS is a technique that can determine the elemental composition of the outermost approximately 3 to 10 nanometers (nm) of a sample surface and that is sensitive to all elements in the periodic table except hydrogen and helium. XPS is a quantitative technique with detection limits for most elements in the 0.1 to 1 atomic percent concentration range. Exemplary surface composition assessment conditions for XPS can include a take-off angle of 90 degrees measured with respect to the sample surface with a solid angle of acceptance of $\pm$ 10 degrees.

When dispersed or suspended in water systems, inorganic materials such as metal silicates exhibit surface charges that are characteristic of the material and the pH of the water system. The potential across the material-water interface is called the "zeta potential," which can be calculated from electrophoretic mobilities (that is, from the rates at which the particles of material travel between charged electrodes placed in the water system). Exemplary concentration agents can have zeta potentials that are more negative than that of, for example, a common metal silicate such as ordinary talc. Yet the concentration agents are surprisingly more effective than talc in concentrating microorganisms such as bacteria, the surfaces of which generally tend to be negatively charged. The concentration agents can have a negative zeta potential at a pH of about 7 (a Smoluchowski zeta potential in the range of about - 9 millivolts to about -25 millivolts at a pH of about 7; a Smoluchowski zeta potential in the range of about -10 millivolts to about -20 millivolts at a pH of about 7; or a Smoluchowski zeta potential in the range of about -11 millivolts to about -15 millivolts at a pH of about 7).

Useful metal silicates include, but are not limited to, amorphous silicates of metals such as magnesium, calcium, zinc, aluminum, iron, titanium, and the like, and combinations thereof. Magnesium, zinc, iron, titanium, or combinations thereof can be utilized. Amorphous metal silicates in at least partially fused particulate form can be utilized. Amorphous, spheroidized metal silicates can be utilized. Amorphous, spheroidized magnesium silicate can be utilized. Metal silicates are known and can be chemically synthesized by known methods or obtained through the mining and processing of raw ores that are naturally-occunring.

Some amorphous metal silicates are commercially available. For example, amorphous, spheroidized magnesium silicate is commercially available for use in cosmetic formulations (for example, as 3M Cosmetic Microspheres CM-111, available from 3M Company, St. Paul, MN).

In addition to amorphous metal silicates, the concentration agents can also include other materials including oxides of metals (for example, iron or titanium), crystalline metal silicates, other crystalline materials, and the like, provided that the concentration agents have the above-described surface compositions. In an embodiment, a concentration agent contains essentially no crystalline silica.

The concentration agents can be used in any form that is amenable to sample contact and microorganism capture. The concentration agents can be used in particulate form. The concentration agent can be in the form of microparticles. The concentration agent can be in the form of microparticles having a particle size in the range of about 1 micrometer (in an example, about 2 micrometers) to about 100 micrometers (in an example, about 50 micrometers; in another example, about 25 micrometers; in yet another example about 15 micrometers; where any lower limit can be paired with any upper limit of the range).

As exemplified in **FIG. 1,** the concentration agent can be added to the receptacle before the sample is added to the sample, path **141,** or after the sample is added to the receptacle, path **143.** The concentration agent can also be added substantially simultaneously with the addition of the sample. Furthermore, in an example (not depicted in **FIG. 1**), the receptacle can be obtained with the concentration agent already contained therein and therefore, a separate step to add the concentration agent would not be necessary. The concentration agent can be added to the receptacle (if necessary) using known techniques. For example, the concentration agent can simply be added to the receptacle or can be added to the receptacle in combination with another component (for example, a liquid). In an example, the concentration agent is simply added to the receptacle (either before, after, or simultaneous with the sample addition) on its own.

The amount of concentration agent added to the receptacle can depend at least in part on the type of concentration agent utilized, the sample size, the receptacle type and size, sample mixing, the particular application, other factors not specifically discussed herein, or a combination thereof. The capture efficiency (the percent of microorganisms in the sample bound to concentration agent) can generally be increased by allowing increased time for the microorganism to come in contact with the concentration agent. The capture efficiency can also be increased by having a higher concentration of concentration agent, which decreases the mean diffusion distance a microorganism must travel to be captured, leading to a shorter incubation time. Therefore, as a generality, the more concentration agent added, the shorter incubation time necessary to capture the same amount of microorganisms.

An appropriate amount of concentration agent can vary given the time necessary to wait for the microorganisms to be bound to the concentration agent (referred to as "capture time"). For example, for a capture time of I minute, 1000 mg of concentration agent per 10 mL of sample could be appropriate; for a capture time of 10 minutes, 100 mg of concentration agent per 10 mL of sample could be appropriate; and for a capture time of 60 minutes, 10 mg of concentration agent per 10 mL of sample could be appropriate. In an example, from about 1 mg to about 100 mg of concentration agent per 10 mL of sample can be utilized. In an example, from about 1 mg to about 50 mg of concentration agent per 10 mL of sample can be utilized. In an example, from about 10 mg to about 25 mg of concentration agent per 10 mL of sample can be utilized. In an example utilizing a metal silicate concentration agent for example, about 10 mg of a metal silicate concentration agent per 10 mL of sample can be utilized. In an example utilizing a metal silicate concentration agent for example, about 25 mg of a metal silicate concentration agent per 10 mL of sample can be utilized.

As discussed above, once the sample and concentration agent is in the receptacle, a microorganism-bound composition (concentration agent-bound microorganisms and sample matrix) can be formed in the receptacle. The next step in the method, as depicted in **FIG. 1** is step **130,** filtering the sample. The term "filtering" is generally used to describe the process of separating matter by size, charge and/or function. For example, filtering can include separating soluble matter and a solvent (e.g., diluent or sample matrix) from insoluble matter, or it can include separating soluble matter, a solvent and relatively small insoluble matter from relatively large insoluble matter. In an example, filtering separates the concentration agent-bound microorganisms from the sample matrix. The step of filtering generally functions to collect the concentration agent-bound microorganisms on the filter and allow the sample matrix to permeate the filter and either be collected or thrown away.

A "filter" is generally used to describe the device used to separate the soluble matter (or soluble matter and relatively small insoluble matter) and solvent from the insoluble matter (or relatively large insoluble matter) in a liquid composition. In an example, a filter separates the sample matrix from the concentration agent-bound microorganisms. Examples of filters can include, but are not limited to, a woven or non-woven mesh (e.g., a wire mesh, a cloth mesh, a plastic mesh, etc.), a woven or non-woven polymeric web (e.g., comprising polymeric fibers laid down in a uniform or nonuniform process, which can be calendered), a sieve, glass wool, a frit, filter paper, foam, etc., and combinations thereof.

Exemplary filters having pore sizes greater than or equal to 1 micrometer are commercially available from numerous sources, examples of commercially available filters include, but are not limited to filters available from 3M CUNO, General Electric Company, Millipore, and Pall Corporation. Exemplary filter membranes with a 10 micrometer pore size are commercially available from GE Osmonics Lab Store (for example GE polycarbonate membrane) and Pall Corporation (MMM-Asymmetric Super-Micron membrane). Exemplary filters can also be prepared as disclosed in commonly assigned U.S. Patent No. 7,553,417, entitled, "FUNCTIONALIZED SUBSTRATES", and PCT Publication No. WO2009/048743, entitled "MICROPOROUS MEMBRANES HAVING A RELATIVELY LARGE AVERAGE PORE SIZE AND METHODS OF MAKING THE SAME".

A filter can be described by its pore size (for example by its bubble point pore size). The bubble point pore size of a filter is generally the average of the largest size of the pores of the filter. In an example, a filter having an average pore size that is greater than the average pore size of the microorganisms is utilized. In an example, the filter can have an average pore size that is less than the average size of the concentration agent. The ability to utilize filters having these relatively large pore sizes offers significant advantages to methods as disclosed herein when compared with other methods for separating microorganisms from samples, such as water samples.

In an example, the filter can have an average pore size that is at least about 1 micrometer ($\mu$m) or larger. In an example, the filter can have an average pore size that is at least about 1.5 $\mu$m or larger. In an example, the filter can have an average pore size that is at least about 5 $\mu$m or larger. In an example, the filter can have an average pore size that is at least about 10 $\mu$m or larger. As larger pore size filters are utilized, the sample will be easier and quicker to filter as the back pressure decreases with increase in pore size.

Filtering the sample can be accomplished using known methods. The method of filtering that is chosen can be dictated at least in part on the particular application of the method. For example, the sample can be filtered using a negative vacuum, by applying a positive pressure, by the force of gravity. The particular technique used to filter the sample can depend at least in part on the type of device that is being utilized to carry out the method. For example, in order to utilize a negative vacuum, the device can be configured with a port that can be or reversibly attached to a source of vacuum; and in order to apply a positive pressure, the device can be configured to allow a user to apply a positive pressure by applying a force with their hands. The sample can be filtered by applying a positive pressure. Filtering using positive pressure (or using the force of gravity) can offer the advantage of easily being able to carry out the method in the field without the need for any further equipment, such as a vacuum pump.

FIG. 2 depicts another exemplary method as disclosed herein. This exemplary method includes the steps of providing a receptacle (step 210), adding sample to the receptacle (step 220), optionally adding concentration agent to the receptacle (step 240), filtering the sample (step 230) and detecting the microorganisms (step 250). In an embodiment, detecting the microorganisms includes identifying the microorganisms, quantifying the microorganisms, or both.

A variety of methods can be used to identify and/or quantify microorganisms, including, but not limited to, microbiological assays, biochemical assays (e.g. immunoassay), nucleic acid analysis, or a combination thereof. Specific examples of testing methods that can be used include, but are not limited to, lateral flow assays, titration, thermal analysis, microscopy (e.g., light microscopy, fluorescent microscopy, immunofluorescent microscopy, scanning electron microscopy (SEM), transmission electron microscopy (TEM)), spectroscopy (e.g., mass spectroscopy, nuclear magnetic resonance (NMR) spectroscopy, Raman spectroscopy, infrared (IR) spectroscopy, x-ray spectroscopy, attenuated total reflectance spectroscopy, Fourier transform spectroscopy, gamma-ray spectroscopy, etc.), spectrophotometry (e.g., absorbance, fluorescence, luminescence, etc.), chromatography (e.g., gas chromatography, liquid chromatography, ion-exchange chromatography, affinity chromatography, etc.), electrochemical analysis, genetic techniques (e.g., polymerase chain reaction (PCR), transcription mediated amplification (TMA), hybridization protection assay (HPA), DNA or RNA molecular recognition assays, etc.), adenosine triphosphate (ATP) detection assays, immunological assays (e.g., enzyme-linked immunosorbent assay (ELISA)), cytotoxicity assays, viral plaque assays, techniques for evaluating cytopathic effect, culture techniques such as those that can be done using a growth medium (e.g., agar) and/or 3M PETRIFILM Plates (e.g., and imaged, quantified and/or interpreted using a 3M PETRIFILM Plate Reader (3M Company, St. Paul, MN)), other suitable analyte testing methods, or a combination thereof. The microorganisms can be detected by culturing the microorganisms and counting the colonies. The microorganisms can be detected colorimetrically, electrochemically, fluorimetrically, or lumimetrically. The microorganisms can be detecting by culturing, or by utilizing immunoassay, enzyme assays or genetic analysis

Microbiological analyses can be conducted immediately after the sample has been collected and concentrated. After the sample has been filtered, the filter, which contains the microorganisms can be removed for microbiological analysis. The filter can be placed into a device with semisolid microbiological culture medium. Nonlimiting examples of such devices include Petri dishes containing various agar media, Petri dishes containing EASYGEL® media (Micrology Laboratories, Goshen IN), and several types of dry, rehydratable culture media, such as 3M PETRIEILM Aerobic Count Plates (3M Company, St. Paul, MN), 3M PETRIFILM Coliform Count Plates, 3M PETRIFILM Colifonn/E.coli Count Plates, COMPACTDRY Total Count Plates (Nissui Pharmaceutical Company, Ltd., Tokyo, JP), SANITA-KUN® Coliforms Plate (Chisso Corporation, Tokyo, JP), and the SANITA-KLTN Total Aerobic Count Plate. In an example, the dry, rehydratable culture media are rehydrated prior to inserting the filter. An advantage of the disclosed method is that the portable, easy to use method can be used with easy to use rehydratable culture media to perform microbiological analyses in a field location with minimal laboratory equipment, such as a small area or glove box for aseptic transfer of the filter onto the culture media. Optionally, a small incubator could provide temperature control for the incubation of the culture media in a field location.

The culture medium can be incubated at an appropriate temperature for an appropriate time subsequent to the placement of the filter onto the culture medium. The appropriate temperature and time for the growth of colonies of microorganisms would be known to a person skilled in the art, and could be in accordance with standard methods. Methods as disclosed herein can be used to concentrate microorganisms that are typically found in water samples for example. Microorganisms that are of particular interest in water samples include, for example, coliforms, fecal coliforms, *Escherichia coli,* and certain species of the genera *Pseudomonas, Aeromonas, Enterococcus, Legionella,* and *Mycobacterium ,* among others.

Tests for coliforms, for example, can be carried out with incubation at a temperature of approximately 35° C for 24 to 48 hours; tests for fecal coliforms can be incubated at a temperature of approximately 45° C. After the period of incubation, the filter can be examined for the presence of bacterial colonies and the number and type of each colony can be recorded. Certain microbiological media, such as Violet Red Bile (VRB) agar contain indicators that distinguish certain bacteria, such as, lactose-fermenting bacteria, from others.

The colonies can be counted manually. When available, devices such as a magnifying lens and/or a dark field magnifying device, such as a Quebec Colony Counter, can be used to assist in counting the colonies. Alternatively the plates can be counted using an automated plate counter such as, for example, ProtoCOL SR or HR colony counting systems from Synbiosis (Fredrick, MD) or a PETRIFILM Plate Reader from 3M Company, provided the filter and the growth medium used in the procedure are compatible with the automated colony counting system.

The filter can be removed from the receptacle, placed on a culture dish (specific for growth of particular bacteria or non-specific for total growth), allowed to grow for a certain period of time and the colonies can be detected by use of bioluminescence reagents and imaging of the plate using an imaging system such as MILLIFLEX® Rapid Microbiology Detection and Enumeration System (Millipore, Bedford, MA).

The sample can be collected and concentrated on the filter and the microorganisms can be directly detected thereon by adding bioluminescent reagents to the filter. Bioluminescence can be quantified by either imaging of the filter or measuring bioluminescence in a luminometer.

The sample can be collected and concentrated on the filter. The filter can optionally be washed, lysis reagents can be added to the filter in order to lyse microorganisms and release the detection analyte such as ATP. The ATP released can be collected and detected by adding bioluminescent reagents to the lysate. Bioluminescence can be quantified by measuring bioluminescence in a luminometer.

The sample can be collected and concentrated and the entire device can be transferred to a laboratory for detection. The sample can be collected and concentrated, the filter can be removed and placed into a sterile container for transport to a laboratory for detection. The container can be designed to keep the filter moist during transport, to avoid loss ofviability of the microorganisms. Optionally, a preservative can be added to the container to maintain the viability of the microorganisms during transport.

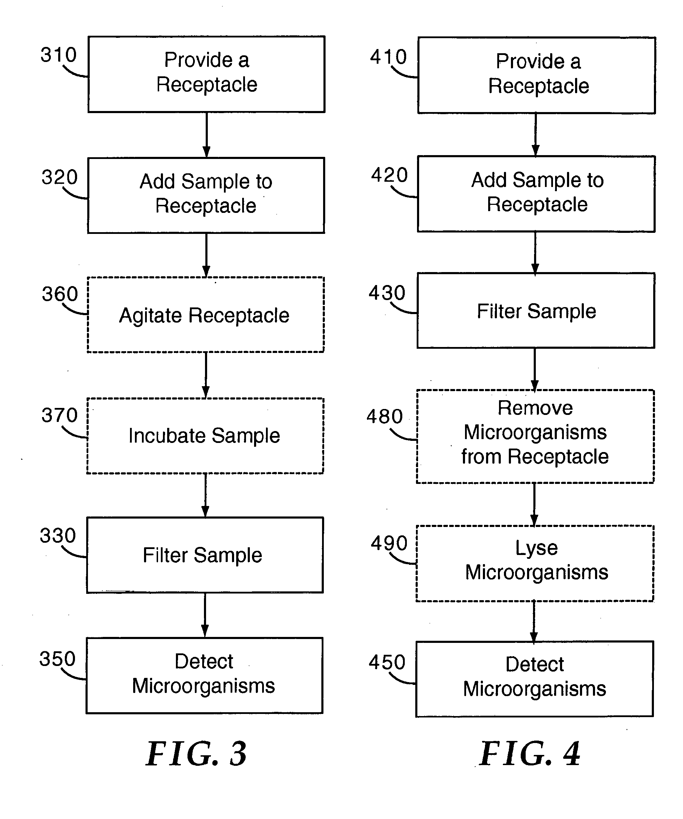

**FIG. 3** depicts another exemplary method disclosed herein. This method includes the steps discussed with respect to **FIG. 2,** but also includes additional optional steps. Step **360** includes agitating the receptacle. The step of agitating the receptacle functions to mix the sample and the concentration agent and can increase microorganism contact with the concentration agent. The term "agitate" and derivatives thereof are generally used to describe the process of giving motion to a liquid composition, for example, to mix or blend the contents of such liquid composition. A variety of agitation methods can be used, including, but not limited to, manual shaking, mechanical shaking (e.g., linear shaking), ultrasonic vibration, vortex stirring, manual stirring, mechanical stirring (e.g., by a mechanical propeller, a magnetic stir bar, or another agitating aid, such as ball bearings), manual beating, mechanical beating, blending, kneading, and combinations thereof.

Agitation may include any of the above-described processes, and for example, can be linear, in a circular orbit, an elliptical orbit, a random orbit, a combination thereof, or of other means to ensure effective and efficient mixing of the sample and the concentration agent. The receptacle may be further secured by clamping or other means during agitation to minimize spillage and/or loss of the contents of the receptacle.

The liquid composition within the receptacle can be agitated by shaking manually by hand. The receptacle containing the liquid composition can be manually shaken for about 15 seconds to about 5 minutes. The receptacle containing the liquid composition can be manually shaken for about 30 seconds to about 3 minutes. The receptacle containing the liquid composition can be manually shaken for about 1 minute to about 2 minutes.

The liquid composition within the receptacle can be agitated by using a Titer Plate Shaker platform (Lab-Line Instruments, Melrose Park, IL). Such an exemplary shaker platform can be operated at settings from 0 to about 10. The shaker platform can be operated at a setting of 2 which is at about 70 rpm. A receptacle containing a liquid composition can be shaken on such an exemplary shaker platform for about 15 minutes to about 2 hours. A receptacle containing a liquid composition can be shaken on such an exemplary shaker platform for about 30 minutes to about 90 minutes. A receptacle containing a liquid composition can be shaken on such an exemplary shaker platform for about 60 minutes (1 hour).

The liquid composition within the receptacle can be agitated by coupling the sample preparation and delivery system 801 to a Burell Model 75 Wrist Action Shaker (Burrell Scientific, Pittsburgh, PA), and agitating at a frequency of 10 to 2000 cycles/minute, and in some embodiments, at a frequency of 200 to 500 cycles/minute for a selected duration of time. The receptacle can be mounted at a distance from the shaker arm from between 5 cm and 50 cm, and in some embodiments, between 10 cm and 20 cm. The receptacle can inscribe an arc of 5 degrees to 30 degrees, and in some embodiments, between 15 degrees and 20 degrees. The liquid composition in the receptacle may be agitated for at least 10 seconds, in some embodiments, at least 15 seconds, in some embodiments, at least 30 seconds, in some embodiments, at least 40 seconds, and in some embodiments, at least 60 seconds. The liquid composition within the receptacle can be agitated for at most 15 minutes, at most 10 minutes, at most 5 minutes, or at most 3 minutes.

The liquid composition within the receptacle can be vortexed in a VX-2500 Multi-Tube Vortexer (VWR Scientific Products, West Chester, PA) at an agitation frequency of 200 to 5000 rpm, or, of 1000 to 3000 rpm for a selected duration of time. The vortex orbit can be linear, circular, elliptical, random, or a combination thereof. In some embodiments, the orbit is between 0.25 cm and 5 cm, and in some embodiments, between 1 cm and 3 cm.

A plurality of receptacles can be agitated simultaneously, by being placed on a plate, an arm or other device, and secured by gravity, clamping or other means for subsequent agitation. For example, one to about fifty receptacles are agitated simultaneously, and in some embodiments, about 10 to about 25 receptacles are agitated simultaneously on a single agitation device or with multiple agitation devices.

Agitation of the liquid composition within the receptacle may be accomplished with steel ball bearings, magnetic stirring bars, blades, and other means to assist in breaking up and/or dispersing the concentration agent within the sample. The agitation methods described above are included by way of example only and are not intended to be limiting. One of ordinary skill in the art will understand that other similar agitation methods can be employed.

**FIG. 3** also includes the optional step of incubating the concentration agent and sample, step **370.** The step of incubating the concentration agent and sample can function to increase microorganism contact with the concentration agent. The concentration agent and sample can be incubated at room temperature or at a controlled temperature. The concentration agent and sample can be incubated at a temperature that is above room temperature. The concentration agent and sample can be incubated at a temperature of about 35° C. The concentration agent and sample can be incubated at a temperature of about 45° C. The time that the concentration agent and sample are incubated can also be varied. The concentration agent and sample can be incubated for about 5 minutes to about 4 hours. The concentration agent and sample can be incubated for about 15 minutes to about 2 hours. The concentration agent and sample can be incubated for about 30 minutes to about 1 hour.

Both step **360** (agitating the sample) and step **370** (incubating the sample) can be carried out on a sample. The step of agitating and the step of incubating can be carried out at the same time. For example, the concentration agent and sample can be shaken for the entire time that the concentration agent and sample are incubated; the concentration agent and sample can be shaken for at least part of the time that the concentration agent and sample are incubated; or the concentration agent and sample can be shaken and then the concentration agent and sample can be incubated.

**FIG. 4** depicts another exemplary method as disclosed herein. The method illustrated in **FIG. 4** includes the steps discussed with respect to **FIG. 2** and also includes optional steps **480** and **490.** Step **480** includes removing at least some of the microorganisms from the receptacle. This can be accomplished by physically removing the filter from the receptacle, by physically removing (e.g. scraping) some of the concentration agent-bound microorganisms from the filter, eluting some of the concentration agent-bound microorganisms from the filter, eluting some of the microorganisms from the concentration agent on the filter, or a combination thereof. In an example, the filter is removed from the receptacle, by for example, use of forceps to grasp the filter and remove it from the receptacle. One of skill in the art will understand, having read this specification how removal of the microorganisms can be accomplished and the circumstances under which it may be desired.

The exemplary method depicted in **FIG. 4** also includes step **490,** lysing the microorganisms. The bound microorganisms can be lysed to render their genetic material available for assay. Lysis methods are well-known and include, for example, treatments such as sonication, osmotic shock, high temperature treatment (for example, from about 50°C to about 100°C), and incubation with an enzyme such as lysozyme, glucolase, zymolose, lyticase, proteinase K, proteinase E, and viral enolysins. One of skill in the art would know, having read this specification, when the microorganisms should be lysed in order to detect them.

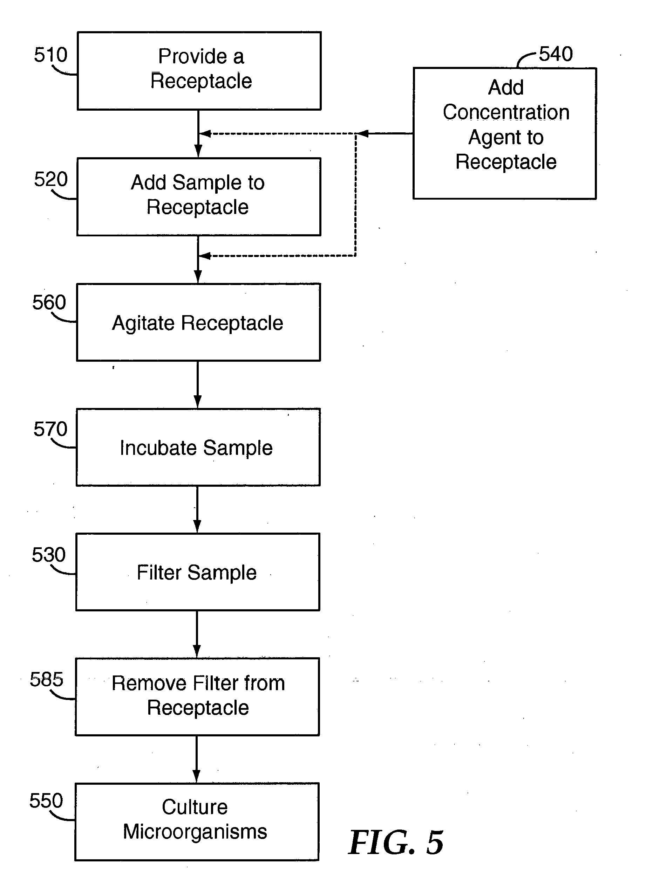

**FIG. 5** depicts another example of a method as disclosed herein. This exemplary method includes the steps of providing a receptacle (step **510**), adding sample to the receptacle (step **520**), adding concentration agent to the receptacle (step **540)** either before, after or at the same time as the sample is added to the receptacle, agitating the receptacle including the sample and concentration agent (step **560**), incubating the concentration agent and sample (step **570**), filtering the sample (step **530**), removing the filter from the receptacle (step **585**) and culturing the microorganisms (step **550**). Such an exemplary method can be useful when the quantity of microorganisms in the sample is to be determined. This exemplary method provides a simple method that can be carried out in the field by a relatively non-skilled technician.

Further methods for isolating microorganisms from a sample containing sample matrix and microorganisms are also disclosed herein that include the steps of placing a liner, which functions as the receptacle, having an opening in a container to form a receptacle assembly, the container configured to at least partially receive the liner; adding the sample to the liner to form a microorganism-bound composition in the liner, the microorganism-bound composition including concentration agent-bound microorganisms and sample matrix; placing a filter over at least a portion of the opening of the liner; placing a filter support on the filter to form a filter assembly, the filter assembly including the liner, the container, the filter and the filter support; inverting the filter assembly; and filtering the microorganism-bound composition through the filter to collect the concentration agent-bound microorganisms on the filter, during which time the liner is deformed or collapses.

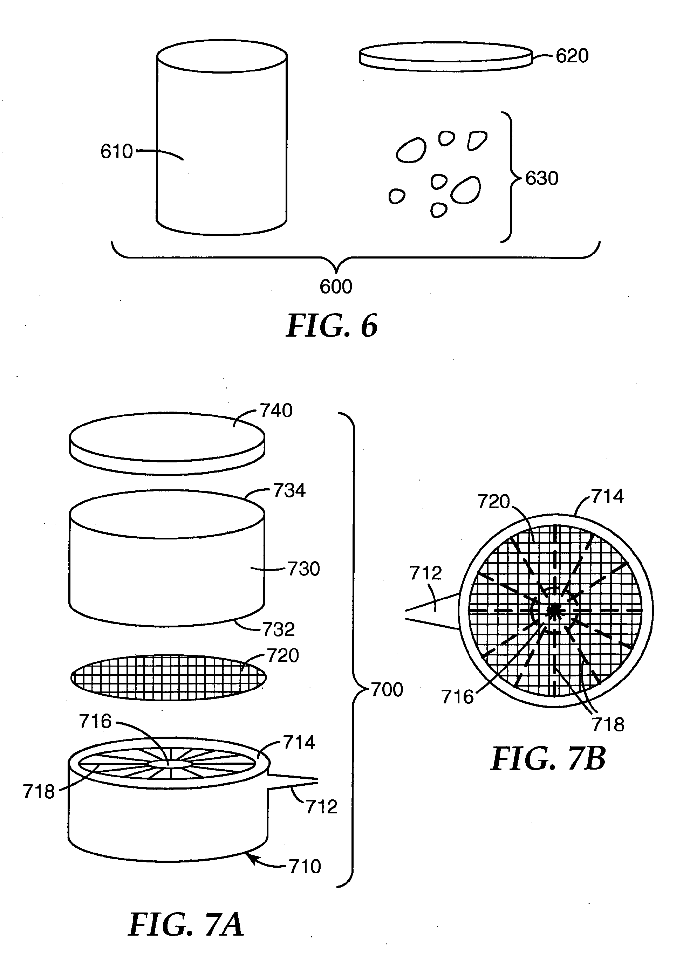

Kits are also disclosed herein. An exemplary kit **600** is illustrated in **FIG. 6** and includes a receptacle **610,** a filter **620** and concentration agent **630**.

General characteristics of receptacles that can be utilized as the receptacle **610** were discussed above. Specific configurations and types of receptacles will also be discussed further below. A kit as disclosed herein can include one or more than one receptacle.

General characteristics of filters that can be utilized as the filter **620** were also discussed above. In an example, a filter has a first surface and a second surface and can generally be planar. As stated above, exemplary filters can include, but are not limited to, a woven or non-woven mesh (e.g., a wire mesh, a cloth mesh, a plastic mesh, etc.), a woven or non-woven polymeric web (e.g., comprising polymeric fibers laid down in a uniform or nonuniform process, which can be calendered), a sieve, glass wool, a frit, filter paper, foam, etc., and combinations thereof. In an example, a filter having an average pore size that is greater than the average pore size of the microorganisms is utilized. In an example, the filter has an average pore size that is smaller than the average size of the concentration agent included in the kit. In an example, filters having average pore sizes that are at least about 1 $\mu$m can be utilized. In an example, filters having an average pore size of about 10 $\mu$m can be utilized. The ability to utilize such large filter sizes can offer the advantage of being able to filter samples relatively quickly without additional equipment.

General characteristics of concentration agents that can be utilized as the concentration agent **630** were also discussed above. Exemplary concentration agents include, but are not limited to particulate or dispersed gamma-FeO (OH), diatomaceous earth that can be (but need not be) surface-treated with titanium dioxide or fine-nanoscale gold or platinum, metal silicates, or combinations thereof. Generally, processing of a water sample having a volume of about 100 mL can be accomplished with about 10 mg to about 100 mg of concentration agent.

Kits can include one or a plurality of filters, one or a plurality of receptacles, an amount of concentration agent sufficient to process one or a plurality of samples. Kits can also include other optional components. In an example, a kit can include components that can be utilized to detect microorganisms. If desired, one or more additives (for example, lysis reagents, bioluminescence assay reagents, nucleic acid capture reagents (for example, magnetic beads), microbial growth media, buffers (for example, to moisten a solid sample), microbial staining reagents, washing buffers (for example, to wash away unbound material), elution agents (for example, serum albumin), surfactants (for example, Triton™ X-100 nonionic surfactant available from Union Carbide Chemicals and Plastics, Houston, TX), mechanical abrasion/elution agents (for example, glass beads), and the like) can be included in a kit as disclosed herein.

An exemplary kit includes concentration agent; and a system for isolating microorganisms from a sample, the system includes a receptacle configured to allow filtering of the sample and to reversibly contain the sample and a concentration agent; and a filter having pores with an average pore size that is larger than the average size of the microorganisms.

One type of exemplary device that can be utilized for carrying out a method as disclosed herein is a vacuum device. An exemplary vacuum device is depicted in **FIG. 7A**. The vacuum device **700** includes a filtrate compartment **710,** a filter **720** and a sample compartment **730**. The sample compartment **730** is an example of a double opening receptacle. The filtrate compartment **710** includes a vacuum port **712,** which allows it to be operably coupled to a source of vacuum. The filtrate compartment **710** communicates with the remainder of the vacuum device **700** via the filtrate channel **716**. The filtrate compartment **710** also includes a filter support, which is made up of a ledge **714** on the outer periphery and a filter support structure **718** that begins at the ledge **714** and terminates at the filtrate channel **716**. The filter support structure **718** functions to provide support to the filter **720** when the vacuum device **700** is operably configured.

During usage, the filter **720** can be placed on the filter support structure **718** and centered on the ledge **714**. The sample compartment **730** is then disposed adjacent to the filter **720** and the sample can be added thereto. The sample can be agitated, incubated, or both with the concentration agent in the sample compartment and then a vacuum can be applied to the filtrate compartment **710** via the vacuum port **712** to cause the concentration agent-bound microorganisms to be filtered from the sample matrix. This will cause the concentration agent-bound microorganism to be collected on the filter **720** and the sample matrix to be collected in the filtrate compartment **710**. The sample compartment **730** can be covered, or sealed using a sample cover **740** to minimize or stop spillage of the sample when agitated or transported for example.

An exemplary device that is similar to the device depicted in **FIG. 7A** and **7B** is a Nalgene Disposable Sterile Filter Unit; membrane, gridded, CN; capacity, 150mL; pore size, 0.45$\mu$m, which can be obtained from numerous vendors, including but not limited to, Cole-Parmer (Vernon Hills, IL) as product number EW-06730-04. This particular product has a sample container made of polypropylene and a filtrate container made of high impact polystyrene; has ¼" and 3/8" inner diameter quick-disconnect tubing adapters for vacuum connection; and utilizes a 47 mm diameter filter. Such an exemplary device (along with an appropriate filter(s)) could be utilized along with concentration agent in a kit or for carrying out the methods disclosed herein.

Another type of exemplary device that can be utilized to carry out a method as disclosed herein is a positive pressure device. One exemplary positive pressure device that can be utilized includes the device described in the commonly assigned PCT Publication No. WO2008/150779, entitled "DEVICES AND PROCESSES FOR COLLECTING AND CONCENTRATING SAMPLES FOR MICROBIOLOGICAL ANALYSIS", cited herein.

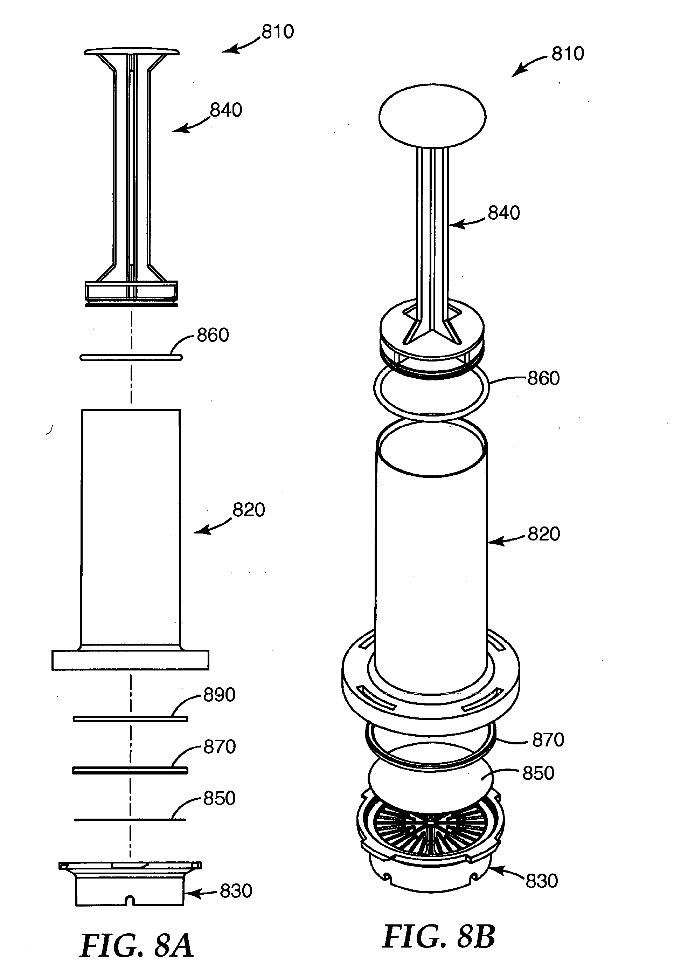

An example of this device is depicted in **FIG. 8A** and **8B**. **FIG. 8A** and **FIG. 8B** show exploded views of the component parts of an exemplary device **810**. The device **810** comprises a hollow, elongated body **820,** which attaches to a removable support **830**. The elongated body **820** is an example of a double opening receptacle. A plunger **840** is shaped and proportioned to fit within and move longitudinally through the interior of the body **820**. The removable support **830**, onto which a filter **850** can be placed, is detachably attached to the body **820**. At the lower end of the plunger **840** there is a sealing ring **860**. At the lower end of the body **820** there is a sealing gasket **870**. The sealing ring **860** and the sealing gasket **870** keep the device **810** sealed to prevent leakage during its use and may be, where appropriate, produced from elastomeric materials, such as thermoplastic elastomers commercialized by ADVANCED ELASTOMER SYSTEMS (based in Akron, Ohio, United States) under the commercial name SANTOPRENE™; acrylonitrile and butadiene copolymers, also known as buna N and commercialized by GOODYEAR TIRE & RUBBER CO. (based in Akron, Ohio, USA) under the commercial name CHEMIGUM™; or silicon rubbers, such as rubber commercialized by DOW SCORNING (based in Midland, Michigan, USA). In certain examples, an optional prefilter **890** as shown in **FIG. 8A,** can be positioned in the device **810** in a location  that is upstream in the flow path, relative to the filter **850**. The filter **850** can be made of materials and have characteristics as discussed above with respect to exemplary filters.

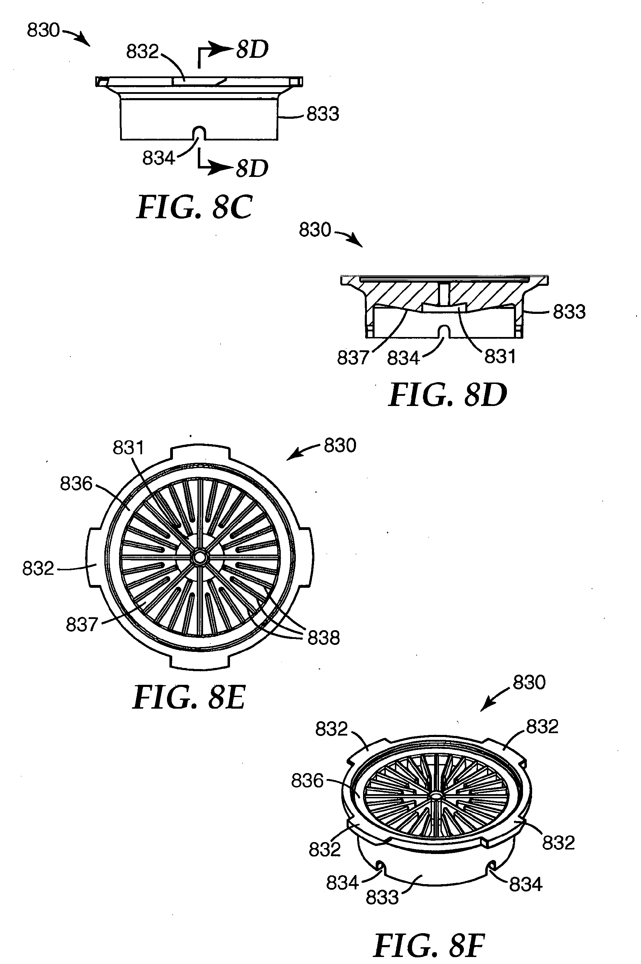

**FIGS. 8C-F** show details of an exemplary removable support **830**. The removable support **830** is attached to the body **820** of the device **810** in a manner such that it can be detached from the same. The structures or methods used for the removable attachment and detachment are generally mechanical and may have diverse configurations (not shown) such as, for example, through pins and balls, mechanical fixing systems of the hook and loop type, bolt and screw systems. Other suitable structures for enabling the body **820** and the removable support **830** to be removably affixed to each other are known in the art, and may be used with this exemplary device. In some examples, the removable support **830** possesses projections **832** which may be fitted into projection clamps **828** on the base **824** of the body **820**. The removable support **830** also presents a filtrate drain **831** for discharge of the filtered matter, after is has passed through the filter **850**. In some examples as shown in **FIGS. 8C, 8D,** and **8E,** the removable support comprises a drain housing **833** and optional drain holes **834** to facilitate the passage of liquid and/or air from the drain housing **833**. The drain hole **834** can be configured to have a variety of shapes, numbers, and sizes. The drain holes **834** provide an egress for filtrate when a removable support **830** similar to that shown in **FIG. 8A** is placed on an essentially smooth, flat surface during the use of device **810.**

Prior to using the device **810** to concentrate a liquid sample, a filter **850** is placed on or in an example, inside the removable support **830** (as shown in **FIG. 8A-B**). In an example, the filter **850** is supported essentially perpendicular to the liquid flow by a shelf **836** and crossbars **838** formed on the internal wall of the removable support **830,** although any suitable arrangement by which liquid can be directed through the filter may be used. The shelf **836** and crossbars **838** essentially form a porous support structure to position a filter in a liquid flow path. The thickness and diameter of the filter **850** should be compatible with the diameter of the shelf **836** of the removable support **830** and the diameter of the sealing gasket **870**. In the assembled device **810,** the sealing gasket **870** is preferably positioned on top of the outer rim of the filter **850**, forming an essentially watertight seal between the filter **850** and the sealing rim **829**.

In the illustrated example, the removable support **830** includes crossbars **838** which project longitudinally downward from the upper end of the removable support **830** to a floor **837** and extend radially inward from the shelf **836**. In this embodiment, the floor **837** has a central opening, the filtrate drain **831,** which helps to direct the liquid flow out of the removable support **830,** as shown in **FIG. 8E**. As shown in **FIGS. 8E** and **8F,** the shelf **836** has the form of a solid ring inside the removable support **830** and, in conjunction with the sealing gasket **870** and the sealing rim **829** of the body **820,** the shelf **836** aids in forming a watertight seal.

The crossbars **838** provide a porous support structure for the filter **850** during the use of the device **810**. Although shown as crossbars **838** in **FIG. 8A-B,** the porous support structure may have various other configurations. An alternative design (not shown) may include a single support member consisting of an essentially solid, preferably planar surface, with a plurality of orifices, which provide passages, for liquid flow out of the removable support, spaced across the surface. The porous support structure provides sufficient support for the filter **850,** so that the filter does not break or pass through the orifices in the porous support structure during the process in which hydrostatic pressure is applied to the filter **850**. The removable support **830** and its components may be produced, in an appropriate form, from polymeric materials. Examples of such materials include, but are not limited to, polypropylene, polyethylene, polyester and polycarbonate.

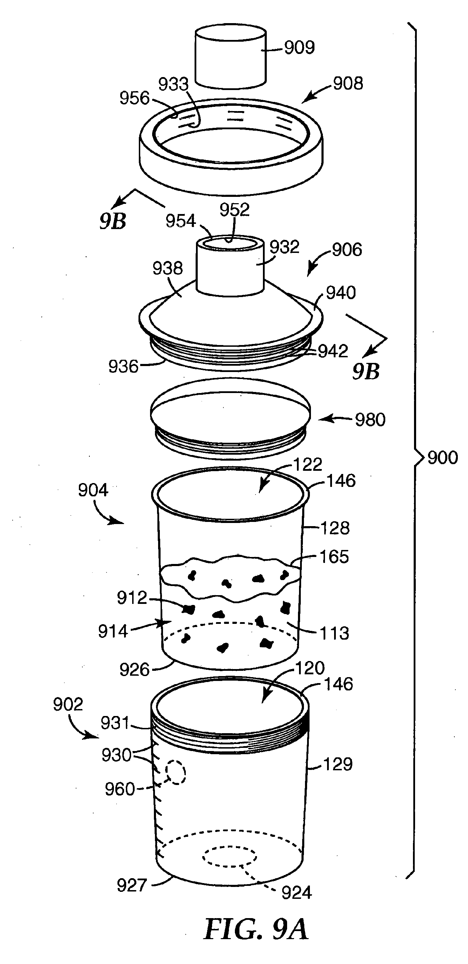

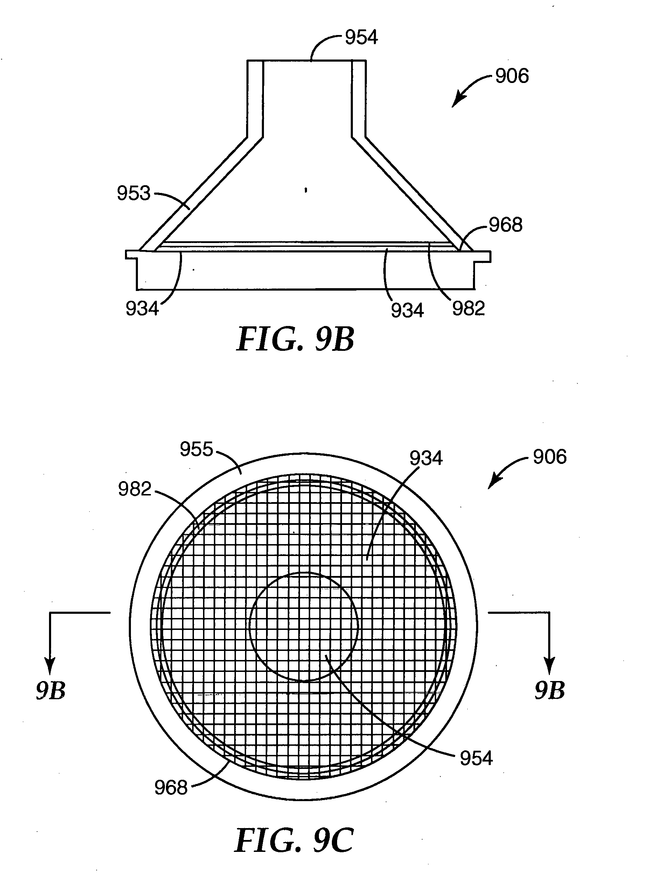

Another exemplary positive pressure type of device can be seen in **FIG. 9**. As shown in **FIG. 9,** the sample preparation system **900** includes a container **902**, a liner **904**, a ring **980**, a lid **906**, a collar **908**, and a cover **909**. The system also includes a filter, but is cannot be seen in the view of **FIG. 9A**. In such an example, the liner **904** is an example of a single opening receptacle.

A system having similar features to that of the sample preparation system **900** is described in PCT Publication No. WO 2009/067503, entitled "SAMPLE PREPARATION FOR ENVIRONMENTAL SAMPLING"; PCT Publication No. WO 2007/137257, entitled "SYSTEM AND METHOD FOR PREPARING SAMPLES" ; each cited herein.

In some examples, as shown in **FIG. 9,** the container **902** is freestanding and/or self-supporting and includes a base **927** and a sidewall **929**. The term "freestanding" is generally used to refer to an object that is capable of standing on its own without collapsing or distorting, and without being held by another object. The term "self-supporting" is generally used to refer to an object that does not collapse or deform under its own weight. For example, a bag is typically not "self-supporting" in that it does not maintain its shape, but rather collapses or distorts, under its own weight. A self-supporting object is not necessarily freestanding.

The container **902** can be formed of a variety of materials including, but not limited to, polymeric materials, metals (e.g., aluminum, stainless steel, etc.), ceramics, glasses, and combinations thereof. Examples of polymeric materials can include, but are not limited to, polyolefins (e.g., polyethylene, polypropylene, combinations thereof, etc.), polycarbonate, acrylics, polystyrene, high density polyethylene (HDPE), polypropylene, other suitable polymeric materials capable of forming a freestanding and/or self-supporting container, or a combination thereof. The container **902** can be translucent (or even transparent), or opaque, and can be any suitable size, depending on the type, amount and size of source to be analyzed. For example, in some embodiments, the container **902** can have a capacity of 50 mL, 100 mL, 250 mL, or larger.

In some examples, as shown in **FIG. 9,** the sample preparation system **900** includes a liner **904,** which is shaped and dimensioned to be received within the container **902**. The liner **904** can be disposable (e.g., made for one-time use), to allow the container **902** to be reused without substantial risk of contamination and without extensive cleaning required between uses. As described in greater detail a sample preparation system can include a liner without a container. When the liner is used without a container, it is not functioning as a "liner," per se, and can be referred to generally as a receptacle or container.

As shown in **FIG. 9,** the container **902** defines a first reservoir **920,** and the liner **904** defines a second reservoir **922**. The liner **904** is shaped and dimensioned to be received within the first reservoir **920** of the container **902**. In some examples, a liquid composition **914** can be contained within the first reservoir **920.** In some embodiments, as shown in **FIG. 9,** the liner **904** is employed, and a liquid composition **914** can be contained within the second reservoir **922,** and the liner **904** can be positioned within the first reservoir **920**. Whether added to the first reservoir **920** or the second reservoir **922,** the liquid composition **914** generally includes (once both are added to the receptacle) concentration agent-bound microorganisms **912** and sample matrix **913**. In some examples, the liner **904** is freestanding, and the liner **904** or the container **902** can serve as a freestanding receptacle that can contain the liquid composition **914**.

The liner **904** can be formed of a variety of materials, including a variety of polymeric materials, including, but not limited to, a polyolefin, including, but not limited to polypropylene (e.g., low density polyethylene (LDPE)), polyethylene, and poly(methylpentene), polyamide (e.g., NYLON®), or a combination thereof. In some examples, the liner **904** is formed from a molding process, such as a thermoforming process. The liner **904** can be translucent (or even transparent), or opaque.

In some examples, as illustrated in **FIG. 9,** the liner **904** is freestanding and/or self-supporting, either of which can allow the liquid composition **914** to be loaded into the liner **904** prior to positioning the liner **904** within the container **902**, without the liner **904** collapsing or distorting. In addition, a freestanding and/or self-supporting liner **904** can aid in weighing, sample or concentration agent (or both) addition, transporting, handling, and/or sample removal.

In some examples, the liner **904** is self-supporting and/or freestanding while also being deformable. The term "deformable" is used to refer to a structure that can be altered from its original shape or state by pressure (e.g., positive or negative) or stress. In examples employing a deformable liner **904,** pressure can be applied to the liner **904** to reduce its size from its original (i.e., unstressed) dimensions. Such pressure can be used to promote removal of the liquid composition **914** (or a filtrate thereof) from the liner **904**. In such examples, the liner **904** can serve as a deformable self-supporting receptacle that can contain the liquid composition **914**. In some examples, the deformable self-supporting receptacle is also freestanding.

A system that utilizes a collapsible liner, such as that depicted in **FIG. 9** can offer advantages over other systems because of the ability of the liner to collapse. The collapsible liner can allow for a simpler overall system because a vent to prevent negative pressure (vacuum) or a source of positive pressure is not necessary.

In some examples, as shown in **FIG. 9,** the container **902** includes an aperture **924** formed in its base **927,** through which a user can access the liner **904** to apply pressure to the liner **904** to cause it to deform. Such pressure can be applied directly by hand, or by an additional device, and could be a manual or automated process. The aperture **924** can be shaped and dimensioned according to the desired application of use. In some examples, base **927** of the container **902** is nothing more than the bottom of the sidewall **929,** or a slight inward projection of the sidewall **929,** such that the liner **904** is easily accessible at the bottom of the container **902**. Said another way, in some examples, the aperture **924** of the container **902** defines a majority of the bottom of the container **902** (e.g., a majority of the cross-sectional area of the container **902**), and the base **927** is only a small portion of the container **902** surrounding the aperture **924**. In examples that do not employ the liner **904,** the container **902** need not include the aperture **924**.

In some examples, the liner **904** includes a relatively rigid base **926** and a relatively thin and deformable sidewall **928**, such that when pressure is applied to the base **926** in a direction parallel to the longitudinal axis of the liner **904** (e.g., via the aperture **924** in the container **902**), the liner **904** deforms in the longitudinal direction (e.g., by virtue of the sidewall **928** collapsing rather than the base **926**). Alternatively, or in addition, the base **926** can be thicker than the sidewall **928**. By way of example only, the thickness of the sidewall **928** is at least 50 $\mu$m, in some embodiments, at least 100 $\mu$m, in some examples, at least 150 $\mu$m, and in some examples, at least 200 $\mu$m. In some examples, the thickness of the base **926** is at least 225 $\mu$m, in some examples, 275 $\mu$m, in some examples, at least 300 $\mu$m, and in some examples, at least 350 $\mu$m.

The liner **904** can further include one or more of baffles, pleats, corrugations, seams, joints, gussets, weakened portions (e.g., annular weakened portions), or a combination thereof, which may be incorporated to assist in controlling the deformability of the liner **904**, and/or can further reduce the internal volume of liner **904**. In some examples, the liner **904** can include an accordion-type configuration. In some examples, liner **904** does not include any grooves on its internal surface, particularly, at the internal junction between the base **926** and the sidewall **928**.

In some examples, the liner **904** is deliberately deformed to impart a disruption to the surface geometry of the liner **904**. Such a disrupted surface geometry can assist in the breakup of the source during agitation. For example, in some examples, an obstruction (e.g., a relatively rigid material) can be positioned between the sidewall **928** of the liner **904** and the container **902** to create a different surface geometry in the sidewall **928** of the liner **904.**

As shown in **FIG. 9,** the container **902** can include indicia **930** to indicate the level (i.e., volume) of contents within the container **902**. One example of suitable indicia is described in US Patent No. 6,588,681. Alternatively, or in addition, the liner **904** can include indicia. To enable the use of the indicia **930** on the container **902** and/or the liner **904,** the container **902** and/or the liner **904** can be translucent, or even transparent to afford seeing the liquid composition **914** through the sidewall **929** of the container **902** and/or the sidewall **928** of the liner **904**. The sidewalls **928** and **929** may also bear other types of markings, such as trademarks, brand names, and the like. The indicia **930** can also be provided on a film that is dimensioned to be received within the container **902** or the liner **904** and which can be formed of a material that includes sufficient internal stresses to cause the film to press outwardly (i.e., radially) against an inner surface of the container **902** or the liner **904.**

The system **900** also includes a lid **906**. As shown in **FIG. 9A,** the lid **906** further includes a port **932,** a cylindrical portion **936** that is dimensioned to be received within the liner **904,** and a generally conical (e.g., frusto-conical) portion **938** that extends from the cylindrical portion **936** to the port **932**. At the junction between the cylindrical portion **936** and the conical portion **938,** the lid **906** further includes a lip **940** that extends radially outwardly from the cylindrical portion **936** and the conical portion **938**. The port **932** includes an opening **954** that has an opening inner surface **952**.

As seen in **FIG. 9B,** the inner surface **953** of the lid **906** includes a lower inner circumferential edge **968**. A filter support **982** is coupled to the lower inner circumferential edge **968** and the filter **934** is directly adjacent to the filter support **982**. A filter **934** that is used herein generally includes a first surface and a second surface. The filter support **982** contacts (in an embodiment, directly contacts) the first surface of the filter **934**. The second surface of the filter **934** contacts the sample. The filter support **982** can be coupled to the lid **906** using the same coupling means described above with respect to the lid **906**. The filter support **982** can be permanently or removably coupled to the lid **906**. The degree of coupling between the filter support **982** and the lid **906** may vary depending on a number of factors including, but not limited to, the filter support **982** material, the lid **906** material, the size and texture of the coupled surface area, and the type of coupling means used. For example, if the filter support **982** includes frayed edges, a wider and/or knurled coupling surface area may be used. Such a wider and/or knurled ultrasonic weld may capture frayed edges of the filter support **982**. To minimize the amount of fraying, the filter support **982** can be cut using a laser, which can fuse the edges of the filter support **982**. Because the resulting laser-cut filter support **982** would include a minimum amount of fraying, if any, a narrower coupling area can be used. In some examples, the coupling area extends completely around the outer periphery of the filter support **982**. In some examples, the coupling area can have an average width (i.e., a dimension within the same plane and substantially perpendicular to the outer periphery of the filter support **982**) of up to 5.0 mm, and in some examples, ranging from 1.0 mm to 3.0 mm. Alternatively, the filter support **982** can be integrally formed with the lid **906,** for example, by a molding process.

The filter support **982** can be formed of the same material as the lid **906** or a different material. The filter support **982** may be flexible, or semi-rigid. In some examples, the filter support **982** is formed from a nylon nonwoven or woven fabric, while the lid **906** is an injection molded part formed of a polymer, such as polypropylene. In such examples, the nylon filter support **982** can be coupled to the lid **906** via an ultrasonic welding technique. During ultrasonic welding, at least a portion of the lower inner circumferential edge **968** can melt to mechanically bond the filter support **982**. Since nylon has a higher melting temperature than polypropylene, the nylon filter support **982** can maintain its structural integrity during the ultrasonic welding process. In such examples, at least a portion of the lower inner circumferential edge **968** can enter into a portion of filter support **982,** thereby encapsulating a portion of the filter support **982**.

The filter support 982 can have dimensions and shapes that vary for a given application. The filter support 982 can have any desired shape including, but not limited to, a circular shape, a square shape, a rectangular shape, a triangular shape, a polygonal shape, a star shape, other suitable shapes, and combinations thereof. In the embodiment illustrated in FIGs. 9B and 9C the filter support 982 has a substantially circular shape.

The dimensions of the filter support 982 may vary depending on the size of the lid 906. In some examples, the filter support 982 has a largest dimension (i.e., length, width, or diameter) ranging from 15 mm to 100 mm, although the filter support 982 may have smaller or larger dimensions. For example, the filter support 982 can have a circular shape and a diameter of 56 mm.

The filter 934 can have a total surface area that is greater than a smallest cross-sectional area of the lid 906. In the lid 906, the smallest cross-sectional area is the cross-sectional area of lid opening 954.

In the example illustrated in FIG. 9, the lid 906 is removably coupled to the liner 904, and the collar 908 is employed to further secure the lid 906 to the container 902. For example, in FIG. 9, the container 902 includes threads 931 at the upper end of the outer surface of the sidewall 929, which are shaped and dimensioned for the collar 908 (having internal threads 933 capable of engaging with the threads 931 on the container 902) to be screwed onto the upper end of the container 902. As an alternative to using the collar 908 for securing the lid 906 to the container 902, other coupling means can be employed including clamping and/or any of the other coupling means described below. In some examples, the liner 904 is not employed, and the lid 906 can be coupled directly to the container 902. In such examples, the collar 908 need not be employed. Thus, the lid 906 can form a seal (e.g., a hermetic seal) with either the container 902 or the liner 904. In some examples, the lid 906 and the container 902 (or the lid 906 and the liner 904) are integrally formed or permanently coupled together.

A variety of coupling means can be employed either between the lid 906 and the liner 904, the lid 906 and the container 902, and/or the collar 908 and the container 902 to allow the respective components to be removably coupled to one another, including, but not limited to, gravity (e.g., one component can be set atop another component, or a mating portion thereof), screw threads, press-fit engagement (also sometimes referred to as "friction-fit engagement" or "interference-fit engagement"), snap-fit engagement, magnets, adhesives, heat sealing, other suitable removable coupling means, and combinations thereof. In some examples, the sample preparation system 900 need not be reopened after the liquid composition 914 is added, such that the container 902, the liner 904, the lid 906 and the collar 908 need not be removably coupled to one another, but rather can be permanently or semi-permanently coupled to one another. Such permanent or semi-permanent coupling means can include, but are not limited to, adhesives, stitches, staples, screws, nails, rivets, brads, crimps, welding (e.g., sonic (e.g., ultrasonic) welding), any thermal bonding technique (e.g., heat and/or pressure applied to one or both of the components to be coupled), snap-fit engagement, press-fit engagement, heat sealing, other suitable permanent or semi-permanent coupling means, and combinations thereof. One of ordinary skill in the art will recognize that some of the permanent or semi-permanent coupling means can also be adapted to be removable, and vice versa, and are categorized in this way by way of example only.

The liner 904 can also include a lip 944 that projects radially outwardly from the sidewall 928 of the liner 904, and which can form an abutting relationship with an upper surface 946 of the container 902 and the lip 940 of the lid 906, such that when the sample preparation system 900 is assembled, the lip 944 of the liner 904 is positioned between the lip 940 of the lid 906 and the upper surface 946 of the container 902, and a seal (e.g., a hermetic seal) is formed. As shown in FIG. 9, the collar 908 includes an inwardly-projecting lip 956, such that when the collar 908 is coupled to the container 902, the lip 956 of the collar 908 presses the lip 940 of the lid 906 into contact with the lip 944 of the liner 904, which is pressed into contact with the upper surface 946 of the container 902 (e.g., to form a higher integrity seal).

A system as disclosed in FIG. 9 can also include an adapter ring 980. The adapter ring 980, if utilized can function to increase the surface area to form a water tight seal between the lid 906 and the liner 904 or container 902. A system 900 can be utilized without an adapter ring 980. The adapter ring 980 is generally configured to fit within the inside surface of the liner 904 and form a larger surface area for contacting the filter 934.

The above-described means for assembling the sample preparation system 900 and for forming a seal between the components of the sample preparation system 900 are described and illustrated by way of example only. One of ordinary skill in the art will understand, however, that a variety of other mechanisms could be employed to assemble the components of the sample preparation system 900 and to form a seal (e.g., a liquid-tight seal, a hermetic seal, or a combination thereof), such that the sample preparation system 900 is inhibited from leaking under normal operating conditions.

While the lid 906 of the embodiment illustrated in FIGs. 9A, 9B and 9C is illustrated as having a generally conical or frusto-conical shape. It should be understood that the lid 906 could have a variety of other shapes, including, but not limited to, a cylindrical shape, a tubular shape having a rectangular or square cross-sectional area, or other shapes suitable to being coupled to the other components of the sample preparation system 900. Similarly, the container 902, the liner 904, and the collar 908 could have a variety of other shapes than the substantially cylindrical shapes illustrated in FIGs. 9A, 9B and 9C. In addition, the lid 906 can be dimensioned to accommodate the other components of the sample preparation system 900.

The lid **906** can be formed of a variety of materials, including the materials listed above with respect to the container **902**. The lid **906** can be translucent (or even transparent), or opaque, depending on the application of use.

The collar **908** can be formed of a variety of materials, including, but not limited to a variety of polymeric materials, metal materials, and combinations thereof. For example, the collar **908** can be formed of a molded plastic component, or a machined metal (such as aluminum) component. In some embodiments, the collar **908** is formed of a molded plastic component comprising glass fiber reinforced polypropylene.

As shown in **FIG. 9A,** the port **932** of the lid **906** is generally cylindrical and tubular in shape, such that the port **932** defines a portion **925** of the inner surface **953** of the lid **906** and an opening **954** in the lid **906**. The lid **906** is hollow and is in fluid communication with the second reservoir **922** when the sample preparation system **900** is assembled. The port **932** does not need to be cylindrical and can instead take on any shaped necessary for a given application.

In the example shown in **FIG. 9,** the cover **909** is shaped and dimensioned to receive at least a portion of the port **932**. As a result, the cover **909** can be coupled to the port **932** of the lid **906** to close the opening **954** in the lid **906** and to seal (e.g., hermetically seal) the sample preparation system **900** from the environment. The cover **909** can be coupled to the lid **906** using any of the above-described coupling means. The cover **909** can be integrally formed with the lid **906** (e.g., a flip-top snap-on cover), or the cover **909** can be separate from the lid **906** (e.g., a screw-on cover). The cover **909** can be formed of a variety of materials, including the materials listed above with respect to the container **902** or the collar **908**.

One such device can be made by modifying a 3M PPS™ Paint Preparation System, commercially available from 3M Company (St. Paul, MN). Example 2 that follows illustrates an exemplary method for modifying the 3M PPS Paint Preparation System to obtain a system that can be utilized herein.

Devices that utilize the force of gravity to filter the concentration agent-bound microorganisms from the sample matrix can also be utilized herein. The devices described above can function as gravity filtration devices in that a vacuum is not applied, or the sample matrix (filtrate) is not forced through the filter via pressure on the receptacle. Other types of systems (besides vacuum filtration, positive pressure systems and gravity filtration systems) can also be utilized herein.

Also described herein are systems for isolating microorganisms from a sample, the sample including sample matrix and microorganisms, the system including: a liner configured to afford contact of a concentration agent and the sample, to provide a microorganism-bound composition that comprises concentration agent having bound microorganisms and sample matrix; a filter, the filter having a first surface and a second surface and having pores having an average pore size that is larger than the average size of the microorganisms; a filter support configured to contact the first surface of the filter and afford contact of the microorganism-bound composition with the second surface of the filter, wherein the liner and filter support are configured to afford filtration of the microorganism-bound composition through the filter in order to collect the concentration agent-bound microorganisms on the second surface of the filter.

EXAMPLES

All cultures were obtained from The American Type Culture Collection (ATCC, Manassas, VA).

Example 1

A microporous polyvinylidene fluoride (PVDF) film was prepared using a 40 mm twin screw extruder. PVDF polymer pellets (3M/Dyneon 1012) were introduced into the hopper of the extruder. The extruder was set with a screw speed of 150 RPM. The nucleating agent (HYPERFORM® HPN-68L), in powder form, was premixed in a 2 liter batch with the glycerol triacetate diluent (TRIACETIN® glycerol triacetate) with a ULTRA TURRAX® T-25 Basic high shear mixer from IKA Works, Inc. (Wilmington, NC) for a period of about 5 minutes (there is only one speed for the unit) to uniformly distribute the powder in a non-agglomerated, non-gritty, smooth to the touch state and then fed, with additional diluent, by a feeding device into the extruder via a port. The PVDF polymer/diluent/nucleating agent weight ratio was 39.85/60.00/0.15 respectively. The total extrusion rate was about 13.6 kg/hr; the cast speed was 1.6 m/min. The extruder had eight zones with a temperature profile of zones 1 to 8 at 188°C. The uniformly mixed polymer/diluent/ nucleator melt was subsequently pumped through a double-chromed coat-hanger slot film die maintained at 166°C, and cast onto a patterned casting wheel maintained at a wheel temperature of 71°C at a speed of 3.0 meters per minute (m/min) to form a film. The film was washed in-line at a wash station with deionized water and was air dried. The washed film was continuously fed into a length orienter and stretched with an inline film stretch ratio of 1.6 x 2.2 at 132° C. The roll of membrane was evaluated and found to have the following properties: an average film thickness of 1.17 mm; a bubble point pore size of 11.8 $\mu$m; a Gurley resistance to air flow of 0.4 sec/50 cc; and a resistance to water flow of 1.4 sec/ 100 cc. Further details regarding manufacture of this filter can be found in the PCT Publication No. WO 2009/048743, entitled "MICROPOROUS MEMBRANES HAVING A RELATIVELY LARGE AVERAGE PORE SIZE AND METHODS OF MAKING THE SAME", cited herein.

The PVDF filter was then further processed to prepare a hydrophilic polyalkylene glycol di(meth)acrylate functionalized large pore size PVDF membrane by saturating the membrane with a 10 weight percent solution of SR344 (Sartomer Co., Inc., Exton, PA) in methanol. The sample was then irradiated with an electron beam at a dose of 20 kilograys (kGy), rinsed three times with water and placed in water that was heated to 70°C for one hour. Further details regarding processing of this filter can be found in Example 9 of United States Patent No. 7,553,417, entitled, "FUNCTIONALIZED SUBSTRATES", cited herein.

An isolated *E. coli* (ATCC 51813) colony was inoculated into 5 ml BBL Trypticase Soy Broth (Becton Dickinson, Sparks, MD) and incubated at 37 °C for 18-20 hours. This overnight culture at approximately $10^9$ colony forming units/ml (CFU/ml) was diluted in Butterfield's Buffer (pH 7.2, VWR, West Chester, PA). A 1:1000 further dilution from a $10^2$ cfu/ml dilution was done in 100 ml of potable water resulting in a final concentration of 0.1 /ml (10 cfus total).

A device as described in PCT Application No. US2008/064939, entitled "DEVICES AND PROCESSES FOR COLLECTING AND CONCENTRATING SAMPLES FOR MICROBIOLOGICAL ANALYSIS", cited herein, was wiped down with 70% isopropyl alcohol in water solution, allowed to air dry for 30 minutes and then washed with sterile deionized water. After additional air drying for 30 minutes, a 4.4 cm diameter membrane filter of pore size 10 microns was placed onto the removable support of the device. The support was tightened to the body of the device using the lock ring, and the exit port was sealed using a 3.3 cm diameter piece of SCOTCH® packaging tape (3M Company, St. Paul, MN), followed by addition of the spiked sample. 100 milligrams of amorphous, spheroidized magnesium silicate concentration agent (sold as 3M Cosmetic Microspheres, [CM-111], 3M Company, St. Paul, MN) were added. The plunger was placed on top the device to cover it and then the contents in the device were mixed by shaking manually at room temperature (25°C) for 2 minutes.

After mixing, the adhesive barrier seal was removed and pressure was applied by manually pushing down the plunger in the device for approximately5 minutes to capture the amorphous, spheroidized magnesium silicate concentration agent on the filter. During this step the sample was drained out of the device thru an exit port at the bottom of the device. After filtration the device was opened to expose the filter. The filter was removed from the lid using sterile forceps and placed concentration agent side up on 3MPETRIFILM E coli/Coliform Count Plate. The plate was hydrated with 1 ml sterile Butterfield's Buffer, sealed and incubated in a 37°C incubator per manufacturers instructions. A 1:1000 dilution from the initial $10^2$ cfu/ml was plated as control on 3MPETRIFILM E coli/Coliform Count Plate. Following overnight incubation the plate was analyzed for bacterial colonies per manufacturers instructions.

Results (Capture Efficiency) were calculated using the following formula:

$$\text{Capture Efficiency} = \frac{\text{Number of colonies on concentration agent covered filter}}{\text{Total number of colonies in the spiked control}} \times 100$$

Out of a total 8 cfus of *E. coli* spiked in 100 ml, 8 cfus were obtained on the plated amorphous, spheroidized magnesium silicate covered filter, resulting in a capture efficiency of 100%.

Example 2

A 3M PPS Paint Preparation System was obtained and modified as follows. The lid with mesh support was generated using a Dremel cutting wheel to remove the lower support ring from the 3M PPS Paint Preparation System lid. The resulting surface was sanded smooth using 100 grit Wet-or-Dry sandpaper (3M Company, St. Paul, MN). The adapter ring was generated in a similar fashion from a second lid using a Dremel cutting tool to remove the top portion of a 3M PPS Paint Preparation System lid followed by sanding. These modifications allowed the filter to be "pinched" around its perimeter between the smooth surfaces of the lid and adapter ring during device assembly. The final modification was to cut a larger hole in the base of the outer cup to facilitate access to the liner during sample processing. Assembly and use of the device is described below.

A microporous polyvinylidene fluoride (PVDF) film was prepared using a 40 mm twin screw extruder. PVDF polymer pellets (3M/Dyneon 1012) were introduced into the hopper of the extruder. The extruder was set with a screw speed of 150 RPM. The nucleating agent (HYPERFORM® HPN-68L), in powder form, was premixed in a 2 liter batch with the glycerol triacetate diluent (TRIACETIN® glycerol triacetate) with a ULTRA TURRAX® T-25 Basic high shear mixer from IKA Works, Inc. (Wilmington, NC) for a period of about 5 minutes (there is only one speed for the unit) to uniformly distribute the powder in a non-agglomerated, non-gritty, smooth to the touch state and then fed, with additional diluent, by a feeding device into the extruder via a port. The PVDF polymer/diluent/nucleating agent weight ratio was 39.85/60.00/0.15 respectively. The total extrusion rate was about 13.6 kg/hr; the cast speed was 1.6 m/min. The extruder had eight zones with a temperature profile of zones 1 to 8 at 188°C. The uniformly mixed polymer/diluent/ nucleator melt was subsequently pumped through a double-chromed coat-hanger slot film die maintained at 166°C, and cast onto a patterned casting wheel maintained at a wheel temperature of 71°C at a speed of 3.0 meters per minute (m/min) to form a film. The film was washed in-line at a wash station with deionized water and was air dried. The washed film was continuously fed into a length orienter and stretched with an inline film stretch ratio of 1.6 x 2.2 at 132° C. The roll of membrane was evaluated and found to have the following properties: an average film thickness of 1.17 mm; a bubble point pore size of 11.8 $\mu$m; a Gurley resistance to air flow of 0.4 sec/50 cc; and a resistance to water flow of 1.4 sec/100 cc. Further details regarding manufacture of this filter can be found in the PCT Publication No. WO 2009/048743, entitled "MICROPOROUS MEMBRANES HAVING A RELATIVELY LARGE AVERAGE PORE SIZE AND METHODS OF MAKING THE SAME", cited herein.

The PVDF filter was then further processed to prepare a hydrophilic polyalkylene glycol di(meth)acrylate functionalized large pore size PVDF membrane by saturating the membrane with a 10 weight percent solution of SR344 (Sartomer Co., Inc., Exton, PA) in methanol. The sample was then irradiated with an electron beam at a dose of 20 kilograys (kGy), rinsed three times with water and placed in water that was heated to 70°C for one hour. Further details regarding processing of this filter can be found in Example 9 of United States Patent No. 7,553,417, entitled, "FUNCTIONALIZED SUBSTRATES", cited herein.

An isolated *E. coli* (ATCC 51813) colony was inoculated into 5 ml BBL Trypticase Soy Broth (Becton Dickinson, Sparks, MD) and incubated at 37 °C for 18-20 hours. This overnight culture at approximately $10^9$ colony forming units/ml (CFU/ml) was diluted in Butterfield's Buffer (pH 7.2, VWR, West Chester, PA). A 1:1000 further dilution from a $10^2$ cfu/ml dilution was done in 100 ml of potable water resulting in a final concentration of 0.1 /ml (10 cfus total). The liner was placed in the cup, followed by addition of the spiked sample. 100 milligrams of amorphous, spheroidized magnesium silicate concentration agent (sold as 3M Cosmetic Microspheres, [CM-111]) were added. The adapter ring was then placed on the liner followed by placing the PVDF membrane (pore size 10 microns) cut to the outer diameter of the lid (6.6 cm) on the adapter ring. The lid containing the nylon support mesh was placed on the filter and tightened using the lock ring.

The contents were mixed at room temperature (25°C) for 60 minutes on a Titer Plate Shaker platform (Lab-Line Instruments, Melrose Park, IL) at approximately 70 rpm setting. After the incubation the devices were turned upside down, and pressure was applied by manually compressing the inner liner for approximately 1.5 minutes to capture the concentration agent on the filter. During this step the filter was supported by the nylon mesh. After filtration the lid was removed to expose the filter. The filter was removed from the lid using sterile forceps and placed concentration agent side up on Tryptic Soy Agar (TSA) plates and cultured overnight in a 37°C incubator. The plates were analyzed manually for bacterial colonies. A 1:1000 dilution from the initial approximately $10^2$ cfu/ml was plated as control on 3MPETRIFILM E coli/Coliform Count Plate. The plate was incubated in a 37°C incubator overnight the plate was analyzed for bacterial colonies per manufacturers instructions. Colonies on the TSA plates with the plated concentration agent covered filter were counted manually.

The capture efficiency was calculated as shown in Example 1 above. Out of a total 17 cfus (average of n=2, standard deviation 8%) spiked in 100 ml, an average count of 17 cfus was obtained on the plated concentration agent covered filter. An average (n=2) capture efficiency of approximately 100% (standard deviation 11%) was observed.

Example 3

3M PPS Paint Preparation Systems were modified as explained in Example 2 above.

An isolated *E. coli* (ATCC 51813) colony was inoculated into 5 ml BBL Trypticase Soy Broth (Becton Dickinson, Sparks, MD) and incubated at 37 °C for 18-20 hours. This overnight culture at approximately $10^9$ colony forming units/ml (CFU/ml) was diluted in Butterfield's Buffer (pH 7.2, VWR, West Chester, PA). A 1:1000 further dilution from a $10^2$ cfu/ml dilution was done in 100 ml of potable water resulting in a final concentration of 0.1 /ml (10 cfus total). The liner was placed in the cup, followed by addition of the spiked sample. 250 milligrams of various concentration agents were added (see Table 1 for specific concentration agents). The X296-Talc was produced as seen in U.S. Patent No. 6,045,913; and CM-111 in the table refers to 3M Cosmetic Microspheres, [CM-111]. The adapter ring was then placed on the liner followed by placing either a nylon membrane (F150A0A or F150COA 3M CUNO, pore size 1.1-1.4 $\mu$m) or a PVDF membrane (made according to U.S. Patent No. 7,338,692 having a pore size of 1 $\mu$m) cut to the outer diameter of the lid (6.6 cm) onto the adapter ring. The lid containing the nylon support mesh was placed on the filter and tightened using the lock ring.

The contents were mixed at room temperature (25°C) for 60 minutes on a Titer Plate Shaker platform (Lab-Line Instruments, Melrose Park, IL) at approximately 70 rpm setting. After the incubation the devices were turned upside down, and pressure was applied by manually compressing the inner liner for approximately 1.5 minutes to capture the concentration agent on the filter. During this step the filter was supported by the nylon mesh. After filtration, the lid was removed to expose the filter. The filter was removed from the lid using sterile forceps and placed concentration agent side up on Tryptic Soy Agar (TSA) plates and cultured overnight in a 37°C incubator. The plates were analyzed manually for bacterial colonies.

The capture efficiency was calculated as shown in Example 1 above and is reported in Table 1 below. The capture data was obtained from TSA plates on which the retrieved concentration agent on filters had been plated.

Amongst bacterial growth on test plates, only colonies characteristic of *E. coli* (1-1.5mm in diameter, beige, dome shaped) were counted.

**Table 1**

| Concentration Agent | Filter | *E. coli* challenge in 100 mL water | *E. coli* recovered on filter | Capture Efficiency |
|---|---|---|---|---|
| X296-Talc | Nylon F150AOA | 14 cfus | 14 cfus | 100% |
| X296-Talc | PVDF | 14 cfus | 14 cfus | 100% |
| CM-111 | Nylon F150COA | 15 cfus* | 15 cfus | 100% |
| CM-111 | PVDF | 15 cfus* | 16 cfus | 107% |
| None | Nylon F150AOA | 15 cfus* | 11 cfus | 73% |
| None | Nylon F150COA | 15 cfus* | 10 cfus | 67% |
| None | PVDF | 15 cfus* | 12 cfus* | 80% |
| * A standard deviation of 10% for the starting inoculum was observed. | | | | |

Example 4

Filtration rates using modified 3M PPS Paint Preparation Systems as explained in Example 2 were determined. Membrane flow rates were measured using a vacuum filtration apparatus (Air Cadet, Barnant Company, Barrington, IL) set to a vacuum pressure of -10 inches of mercury. The following filters were obtained: Gelman NYLAFLO® 0.2 $\mu$m pore size (obtained from VWR, West Chester, PA); Stratagene Duralon 0.42 $\mu$m pore size (Stratagene, La Jolla, CA); F150AOA or F150COA 3M CUNO, pore size 1.1-1.4 $\mu$m; and a PVDF membrane made according to U.S. Patent No. 7,338,692 having a pore size of 1 $\mu$m. 48 mm disks of the membranes were cut from larger pieces of membrane and placed in the filter housing. The vacuum was turned on and 100 mL of 18 megaohm water (MILLI-Q® Biocel; Millipore; Bedford, MA) was added to the receptacle. The amount of time necessary to filter the sample using each type of filter was measured using a stopwatch. The mean time for each membrane was determined from the average of three replicate samples, as shown in Table 2.

Filtration times for water samples (100 mL) containing 250 mg spheroidized magnesium silicate concentration agent (sold as 3M Cosmetic Microspheres, [CM-111]) and X296-Talc (produced as seen in U.S. Patent No. 6,045,913) were also measured. By increasing the pore size of the membrane, the amount of time required to filter 100 mL samples was decreased. The presence of the particles did not significantly increase the filtration time. Reference membranes typical of those used for filtration of bacteria (pore sizes of 0.2 to 0.5 micrometers) were included in the analysis for comparison. The results can be seen in Table 2 below.

**Table 2**

| Membrane | Mean Pore Size ($\mu$m) | Mean Time to Filter 100 mL (seconds) | | |
|---|---|---|---|---|
| | | No particles | X-296 Talc | CM-111 |
| None | | 4 | | |
| Gelman Nylaflo | 0.2 | 158 | 170 | 152 |
| Stratagene Duralon | 0.42 | 88 | 94 | 92 |
| PVDF | 1.0 | 15 | 23 | 23 |
| F150AOA | 1.5 | 25 | 30 | 28 |
| F150COA | 1.4 | 24 | 31 | 29 |

Thus, embodiments of methods for processing samples are disclosed. One skilled in the art will appreciate that the present disclosure can be practiced with embodiments other than those disclosed. The disclosed embodiments are presented for purposes of illustration and not limitation, and the present disclosure is limited only by the claims that follow.

**Claims**

1. A method for isolating microorganisms from a sample, the sample comprising sample matrix and microorganisms, the method comprising the steps of:

   providing a receptacle, the receptacle configured to allow filtering of the sample and to reversibly contain the sample and a concentration agent;
   wherein the concentration agent comprises a particulate or dispersed material that microorganisms will bind to when the microorganisms are dispersed in a sample;
   wherein the concentration agent includes surface-treated diatomaceous earth, gamma-FeO(OH), or metal silicates;
   adding the sample to the receptacle, wherein a microorganism-bound composition will be formed in the receptacle, the microorganism-bound composition comprising concentration agent-bound microorganisms and sample matrix;
   wherein the microorganism is selected from the group consisting of bacteria, yeast, filamentous fungi, bacterial spores, and fungal spores;
   filtering the microorganism-bound composition through a filter to collect the concentration agent-bound microorganisms on the filter, wherein the filter has an average pore size that is greater than the average size of the microorganisms;
   wherein the average pore size of the filter is smaller than the average size of the concentration agent;
   placing the filter into a device with semisolid microbiological culture medium; and
   culturing the microorganisms on the filter.

2. The method according to claim 1 further comprising adding the concentration agent to the receptacle after the sample is added to the receptacle.

3. The method according to claim 1, wherein the receptacle contains the concentration agent before the sample is added to the receptacle.

4. The method according to claim 1 wherein the step of filtering is accomplished by using a negative vacuum, by applying a positive pressure, or by the force of gravity.

5. The method according to claim 1 further comprising agitating the concentration agent and the sample in the receptacle.

6. The method according to claim 1 further comprising incubating the concentration agent and sample in the receptacle for a period of time before filtering the microorganism-bound composition through the filter.

7. A method for isolating microorganisms from a sample, the sample comprising sample matrix and microorganisms, the method comprising the steps of:

   placing a liner having an opening in a container to form a receptacle assembly, the container configured to at least partially receive the liner;
   adding the sample and a concentration agent to the liner to form a microorganism-bound composition in the liner, the microorganism-bound composition comprising concentration agent-bound microorganisms and sample matrix;
   wherein the concentration agent comprises a particulate or dispersed material that microorganisms will bind to when the microorganisms are dispersed in a sample;
   wherein the concentration agent includes surface-treated diatomaceous earth, gamma-FeO(OH), or metal silicates;
   wherein the microorganism is selected from the group consisting of bacteria, yeast, filamentous fungi, bacterial spores, and fungal spores;
   placing a filter over at least a portion of the opening of the liner, the filter having an average pore size that is greater than the average size of the microorganisms but smaller than the average size of the concentration agent;
   placing a filter support on the filter to form a filter assembly, the filter assembly comprising the liner, the container, the filter and the filter support;
   inverting the filter assembly;
   filtering the microorganism-bound composition through the filter to collect the concentration agent-bound microorganisms on the filter;
removing the filter from the filter assembly after the microorganism-bound composition has been filtered;
placing the filter into a device with semisolid microbiological culture medium; and
culturing the microorganisms on the filter.

8. The method according to claim 7 further comprising adding the concentration agent to the liner before the sample is added to the liner.

9. The method according to claim 7, wherein the concentration agent is present in the liner before the sample is added.

10. The method according to claim 7 further comprising agitating the concentration agent and the sample in the liner.

11. The method according to claim 10 further comprising placing a temporary cover over at least a portion of the opening of the liner before agitating.

12. The method according to claim 7 further comprising incubating the concentration agent and sample for a period of time before filtering the microorganism-bound composition through the filter.

13. The method according to claim 7 wherein filtering further comprises applying pressure on the liner.

**Patentansprüche**

1. Verfahren zum Isolieren von Mikroorganismen aus einer Probe, wobei die Probe Probenmatrix und Mikroorganismen umfasst, wobei das Verfahren die Schritte umfasst:

Bereitstellen eines Aufnahmebehälters, wobei der Aufnahmebehälter dafür gestaltet ist, Filtrieren der Probe zu erlauben und die Probe und ein Konzentrierungsmittel reversibel zu beinhalten;
wobei das Konzentrierungsmittel ein partikelförmiges oder dispergiertes Material umfasst, an das Mikroorganismen binden werden, wenn die Mikroorganismen in einer Probe dispergiert sind;
wobei das Konzentrierungsmittel oberflächenbehandelte Kieselerde, gamma-FeO(OH) oder Metallsilicate enthält;
Zugeben der Proben zu dem Aufnahmebehälter, wobei eine Mikroorganismen-gebundene Zusammensetzung in dem Aufnahmebehälter gebildet werden wird, wobei die Mikroorganismen-gebundene Zusammensetzung Konzentrierungsmittel-gebundene Mikroorganismen und Probenmatrix umfasst;
wobei der Mikroorganismus ausgewählt ist aus der Gruppe bestehend aus Bakterien, Hefe, Fadenpilzen, Bakteriensporen und Pilzsporen;
Filtrieren der Mikroorganismen-gebundenen Zusammensetzung durch ein Filter, um die Konzentrierungsmittel-gebundenen Mikroorganismen auf dem Filter zu sammeln, wobei das Filter eine mittlere Porengröße aufweist, die größer als die mittlere Größe der Mikroorganismen ist;
wobei die mittlere Porengröße des Filters kleiner als die mittlere Größe des Konzentrierungsmittels ist;
Platzieren des Filters in ein Einheit mit einem halbfesten mikrobiologischen Kulturmedium; und
Kultivieren der Mikroorganismen auf dem Filter.

2. Verfahren gemäß Anspruch 1, ferner umfassend Zugeben des Konzentrierungsmittels zu dem Aufnahmebehälter nachdem die Probe zu dem Aufnahmebehälter zugegeben ist.

3. Verfahren gemäß Anspruch 1, wobei der Aufnahmebehälter das Konzentrierungsmittel enthält, bevor die Probe zu dem Aufnahmebehälter zugegeben wird.

4. Verfahren gemäß Anspruch 1, wobei der Schritt des Filtrierens durch Verwendung eines negativen Unterdrucks, durch Anlegen eines positiven Drucks oder durch die Schwerkraft durchgeführt wird.

5. Verfahren gemäß Anspruch 1, ferner umfassend Bewegen des Konzentrierungsmittels und der Probe in dem Aufnahmebehälter.

6. Verfahren gemäß Anspruch 1, ferner umfassend Inkubieren des Konzentrierungsmittels und der Probe in dem Aufnahmebehälter über einen Zeitraum vor dem Filtrieren der Mikroorganismen-gebundenen Zusammensetzung

21

durch das Filter.

7. Verfahren zum Isolieren von Mikroorganismen aus einer Probe, wobei die Probe Probenmatrix und Mikroorganismen umfasst, wobei das Verfahren die Schritte umfasst:

Anordnen einer Auskleidung, die eine Öffnung aufweist, in einem Behälter, um eine Aufnahmebehälter-Baugruppe zu bilden, wobei der Behälter dafür gestaltet ist, die Auskleidung wenigstens teilweise aufzunehmen;
Zugeben der Probe und eines Konzentrierungsmittels zu der Auskleidung, um eine Mikroorganismen-gebundene Zusammensetzung in der Auskleidung zu bilden, wobei die Mikroorganismen-gebundene Zusammensetzung Konzentrierungsmittel-gebundene Mikroorganismen und Probenmatrix umfasst;
wobei das Konzentrierungsmittel ein partikelförmiges oder dispergiertes Material umfasst, an das Mikroorganismen binden werden, wenn die Mikroorganismen in einer Probe dispergiert sind;
wobei das Konzentrierungsmittel oberflächenbehandelte Kieselerde, gamma-FeO(OH) oder Metallsilicate enthält;
wobei der Mikroorganismus ausgewählt ist aus der Gruppe bestehend aus Bakterien, Hefe, Fadenpilzen, Bakteriensporen und Pilzsporen;
Anordnen eines Filters über wenigstens einem Teil der Öffnung der Auskleidung, wobei das Filter eine mittlere Porengröße aufweist, die größer als die mittlere Größe der Mikroorganismen aber kleiner als die mittlere Größe des Konzentrierungsmittels ist;
Anordnen eines Filterträgers auf dem Filter, um eine Filter-Baugruppe zu bilden, wobei die Filter-Baugruppe die Auskleidung, den Behälter, das Filter und den Filterträger umfasst;
Umdrehen der Filter-Baugruppe;
Filtrieren der Mikroorganismen-gebundenen Zusammensetzung durch das Filter, um die Konzentrierungsmittel-gebundenen Mikroorganismen auf dem Filter zu sammeln;
Entfernen des Filters aus der Filter-Baugruppe, nachdem die Mikroorganismen-gebundene Zusammensetzung filtriert worden ist;
Platzieren des Filters in ein Einheit mit einem halbfesten mikrobiologischen Kulturmedium; und
Kultivieren der Mikroorganismen auf dem Filter.

8. Verfahren gemäß Anspruch 7, ferner umfassend Zugeben des Konzentrierungsmittels zu der Auskleidung bevor die Probe zu der Auskleidung zugegeben wird.

9. Verfahren gemäß Anspruch 7, wobei das Konzentrierungsmittel in der Auskleidung vorhanden ist, bevor die Probe zugegeben wird.

10. Verfahren gemäß Anspruch 7, ferner umfassend Bewegen des Konzentrierungsmittels und der Probe in der Auskleidung.

11. Verfahren gemäß Anspruch 10, ferner umfassend Platzieren einer zeitweiligen Abdeckung über wenigstens einem Teil der Öffnung der Auskleidung vor dem Bewegen.

12. Verfahren gemäß Anspruch 7, ferner umfassend Inkubieren des Konzentrierungsmittels und der Probe über einen Zeitraum vor dem Filtrieren der Mikroorganismen-gebundenen Zusammensetzung durch das Filter.

13. Verfahren gemäß Anspruch 7, wobei das Filtrieren ferner Anlegen von Druck an die Auskleidung umfasst.

**Revendications**

1. Procédé pour isoler des micro-organismes à partir d'un échantillon, l'échantillon comprenant une matrice d'échantillon et des micro-organismes, le procédé comprenant les étapes de :

fourniture d'un réceptacle, le réceptacle étant configuré pour permettre la filtration de l'échantillon et pour contenir de façon réversible l'échantillon et un agent de concentration ;
l'agent de concentration comprenant un matériau particulaire ou dispersé auquel les micro-organismes se lient lorsque les micro-organismes sont dispersés dans un échantillon ;
l'agent de concentration comprenant de la terre de diatomées traitée en surface, gamma-FeO(OH), ou des silicates de métal ;

ajout de l'échantillon au réceptacle, une composition liée à des micro-organismes étant formée dans le réceptacle, la composition liée à des micro-organismes comprenant des micro-organismes liés à un agent de concentration et une matrice d'échantillon ;

le micro-organisme étant choisi dans le groupe constitué de bactéries, levures, champignons filamenteux, spores bactériennes, et spores fongiques ;

filtration de la composition liée à des micro-organismes à travers un filtre pour collecter les micro-organismes liés à un agent de concentration sur le filtre, le filtre ayant une taille de pore moyenne qui est supérieure à la taille moyenne des micro-organismes ;

la taille de pore moyenne du filtre étant inférieure à la taille moyenne de l'agent de concentration ;

placement du filtre dans un dispositif avec du milieu de culture microbiologique semi-solide ; et

culture des micro-organismes sur le filtre.

2. Procédé selon la revendication 1 comprenant en outre l'ajout de l'agent de concentration au réceptacle après que l'échantillon a été ajouté au réceptacle.

3. Procédé selon la revendication 1, dans lequel le réceptacle contient l'agent de concentration avant que l'échantillon soit ajouté au réceptacle.

4. Procédé selon la revendication 1 dans lequel l'étape de filtration est effectuée en utilisant un vide négatif, par application d'une pression positive, ou par la force de gravité.

5. Procédé selon la revendication 1 comprenant en outre l'agitation de l'agent de concentration et de l'échantillon dans le réceptacle.

6. Procédé selon la revendication 1 comprenant en outre l'incubation de l'agent de concentration et de l'échantillon dans le réceptacle pendant une période avant la filtration de la composition liée à des micro-organismes à travers le filtre.

7. Procédé pour isoler des micro-organismes à partir d'un échantillon, l'échantillon comprenant une matrice d'échantillon et des micro-organismes, le procédé comprenant les étapes de :

placement d'une cuve ayant une ouverture dans un conteneur pour former un ensemble de réceptacle, le conteneur étant configuré pour recevoir au moins partiellement la cuve ;

ajout de l'échantillon et d'un agent de concentration à la cuve pour former une composition liée à des micro-organismes dans la cuve, la composition liée à des micro-organismes comprenant des micro-organismes liés à un agent de concentration et une matrice d'échantillon ;

l'agent de concentration comprenant un matériau particulaire ou dispersé auquel des micro-organismes se lient lorsque les micro-organismes sont dispersés dans un échantillon ;

l'agent de concentration comprenant de la terre de diatomées traitée en surface, gamma-FeO(OH), ou des silicates de métal ;

le micro-organisme étant choisi dans le groupe constitué de bactéries, levures, champignons filamenteux, spores bactériennes, et spores fongiques ;

placement d'un filtre sur au moins une partie de l'ouverture de la cuve, le filtre ayant une taille de pore moyenne qui est supérieure à la taille moyenne des micro-organismes mais inférieure à la taille moyenne de l'agent de concentration ;

placement d'un support de filtre sur le filtre pour former un ensemble de filtre, l'ensemble de filtre comprenant la cuve, le conteneur, le filtre et le support de filtre ;

retournement de l'ensemble de filtre ;

filtration de la composition liée à des micro-organismes à travers le filtre pour collecter les micro-organismes liés à un agent de concentration sur le filtre ;

retrait du filtre de l'ensemble de filtre après que la composition liée à des micro-organismes a été filtrée ;

placement du filtre dans un dispositif avec du milieu de culture microbiologique semi-solide ; et

culture des micro-organismes sur le filtre.

8. Procédé selon la revendication 7 comprenant en outre l'ajout de l'agent de concentration à la cuve avant que l'échantillon soit ajouté à la cuve.

9. Procédé selon la revendication 7, dans lequel l'agent de concentration est présent dans la cuve avant que l'échantillon soit ajouté.

10. Procédé selon la revendication 7 comprenant en outre l'agitation de l'agent de concentration et de l'échantillon dans la cuve.

11. Procédé selon la revendication 10 comprenant en outre le placement d'une couverture temporaire sur au moins une partie de l'ouverture de la cuve avant agitation.

12. Procédé selon la revendication 7 comprenant en outre l'incubation de l'agent de concentration et de l'échantillon pendant une période avant la filtration de la composition liée à des micro-organismes à travers le filtre.

13. Procédé selon la revendication 7 dans lequel la filtration comprend en outre l'application de pression sur la cuve.

110 — Provide a Receptacle

140 — Add Concentration Agent to Receptacle

141

120 — Add Sample to Receptacle

143

130 — Filter Sample

*FIG. 1*

210 — Provide a Receptacle

240 — Add Concentration Agent to Receptacle

220 — Add Sample to Receptacle

230 — Filter Sample

250 — Detect Microorganisms

*FIG. 2*

310 — Provide a Receptacle

↓

320 — Add Sample to Receptacle

↓

360 — Agitate Receptacle

↓

370 — Incubate Sample

↓

330 — Filter Sample

↓

350 — Detect Microorganisms

*FIG. 3*

410 — Provide a Receptacle

↓

420 — Add Sample to Receptacle

↓

430 — Filter Sample

↓

480 — Remove Microorganisms from Receptacle

↓

490 — Lyse Microorganisms

↓

450 — Detect Microorganisms

*FIG. 4*

510 — Provide a Receptacle

540 — Add Concentration Agent to Receptacle

520 — Add Sample to Receptacle

560 — Agitate Receptacle

570 — Incubate Sample

530 — Filter Sample

585 — Remove Filter from Receptacle

550 — Culture Microorganisms

*FIG. 5*

FIG. 6

FIG. 7A

FIG. 7B

810

840

860

820

890

870

850

830

*FIG. 8A*

810

840

860

820

870

850

830

*FIG. 8B*

830

832

8D

833

834

8D

*FIG. 8C*

830

833

837  834  831

*FIG. 8D*

831

836

832

837

830

838

*FIG. 8E*

830

832

832

836

832

832

834

833

834

*FIG. 8F*

FIG. 9A

FIG. 9B

FIG. 9C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8909279 A1
- WO 2008150779 A1
- WO 2009067503 A
- WO 2007137257 A
- WO 2008150779 A
- WO 2009046191 A
- WO 2009046183 A
- US 4729846 A, Matsui

- WO 2009085357 A
- US 7553417 B
- WO 2009048743 A
- WO 2008150779 PCT
- US 6588681 B
- US 2008064939 W
- US 6045913 A
- US 7338692 B